(19) Europäisches Patentamt
European Patent Office
Office européen des brevets

(11) Veröffentlichungsnummer: **0 370 300 B1**

(12) # EUROPÄISCHE PATENTSCHRIFT

(45) Veröffentlichungstag der Patentschrift: **06.04.94**

(51) Int. Cl.[5]: **C07D 249/08**, C07D 233/60, C07C 255/45, A61K 31/41

(21) Anmeldenummer: **89120575.9**

(22) Anmeldetag: **07.11.89**

(54) **Cyclopropyl-Substituierte Azolylmethylcarbinole, Verfarhren zu ihrer Herstellung und ihre Verwendung als Arzneimittel.**

(30) Priorität: **19.11.88 DE 3839170**

(43) Veröffentlichungstag der Anmeldung:
**30.05.90 Patentblatt 90/22**

(45) Bekanntmachung des Hinweises auf die Patenterteilung:
**06.04.94 Patentblatt 94/14**

(84) Benannte Vertragsstaaten:
**AT BE CH DE ES FR GB GR IT LI LU NL SE**

(56) Entgegenhaltungen:
**EP-A- 0 180 850**
**US-A- 4 859 232**

**Chemical Abstr., Bd. 112, Nr. 19, 07.05.90, S. 773-773, Zusammenfassung Nr.178994w, Columbus, Ohio, US.**

(73) Patentinhaber: **BAYER AG**

**D-51368 Leverkusen(DE)**

(72) Erfinder: **Fugmann, Burkhard, Dr.**
**Ellenbeek 31**
**D-5603 Wülfrath(DE)**
Erfinder: **Plempel, Manfred, Dr.**
**Zwengenbergstrasse 3C**
**D-5657 Haan(DE)**
Erfinder: **Stroech, Klaus, Dr.**
**Rolsberger Strasse 22**
**D-5650 Solingen 19(DE)**
Erfinder: **Büchel, Karl Heinz, Prof. Dr.**
**Dabringhausener Strasse 42**
**D-5093 Burscheid(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die vorliegende Erfindung betrifft neue 1-Cyanocyclopropyl- und 1-Carboxamido-cyclopropyl-azolylmethyl-carbinolderivate, Verfahren zu ihrer Herstellung und ihre Verwendung zur Herstellung von Arzneimitteln, insbesondere von Antimykotika.

Es ist bereits bekannt, daß substituierte Azolylmethylcyclopropyl-carbinol-Derivate antimykotische Eigenschaften aufweisen (vgl. EP-OS 0 180 850). Die Wirkung dieser Stoffe ist jedoch nicht immer in allen Indikationsbereichen voll zufriedenstellend.

Es wurden nun neue 1-Cyano- und 1-Carboxamido-cyclopropyl-azolylmethyl-carbinole der allgemeinen Formel (I) gefunden,

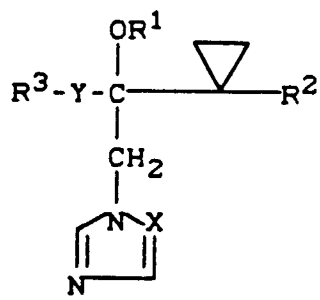

(I)

in welcher

$R^1$

- für Wasserstoff oder
- für Alkyl mit bis zu 10 Kohlenstoffatomen oder
- für Alkylcarbonyl mit bis zu 8 Kohlenstoffatomen steht

$R^2$

- für Cyano oder
- für eine Gruppe der Formel

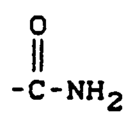

steht

$R^3$

- für Phenyl steht, das gegebenenfalls bis zu 5-fach gleich oder verschieden substituiert ist durch Halogen, geradkettiges oder verzweigtes Alkyl mit bis zu 8 Kohlenstoffatomen, Alkoxy mit bis zu 6 Kohlenstoffatomen, Alkylthio mit bis zu 6 Kohlenstoffatomen, Halogenalkyl, Halogenalkoxy oder Halogenalkylthio mit bis zu 6 Kohlenstoffatomen im Alkylteil und mit bis zu 5 Halogenatomen oder durch Phenyl oder Phenoxy, die ihrerseits durch Halogen oder Alkyl mit bis zu 6 Kohlenstoffatomen substituiert sein können

X

- für ein Stickstoffatom oder für die CH-Gruppe steht

Y

- für eine Bindung oder
- für eine Gruppe der Formel

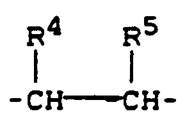

,

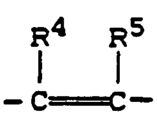

oder -C≡C- steht

worin

$R^4$ und $R^5$ gleich oder verschieden sind und Wasserstoff oder Alkyl mit bis zu 8 Kohlenstoffatomen bedeuten,

sowie deren Säureadditions-Salze.

Bevorzugt sind die erfindungsgemäßen Verbindungen der allgemeinen Formel (I), in welcher

$R^1$

- für Wasserstoff oder
- für Alkyl mit bis zu 8 Kohlenstoffatomen oder
- für Alkylcarbonyl mit bis zu 6 Kohlenstoffatomen steht

$R^2$

- für Cyano oder für eine Gruppe der Formel

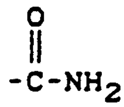

steht

$R^3$

- für Phenyl steht, das gegebenenfalls bis zu 3-fach gleich oder verschieden substituiert ist durch Fluor, Chlor, Brom, geradkettiges oder verzweigtes Alkyl mit bis zu 6 Kohlenstoffatomen, Alkoxy mit bis zu 4 Kohlenstoffatomen, Alkylthio mit bis zu 4 Kohlenstoffatomen, Halogenalkyl, Halogenalkoxy oder Halogenalkylthio mit bis zu 4 Kohlenstoffatomen im Alkylteil und mit bis zu 4 Halogenatomen oder durch Phenyl oder Phenoxy, die ihrerseits durch Fluor, Chlor, Brom oder Alkyl mit bis zu 4 Kohlenstoffatomen substituiert sein können

X

- für ein Stickstoffatom oder für die CH-Gruppe steht

Y

- für eine Bindung oder
- für eine Gruppe der Formel

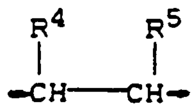 , 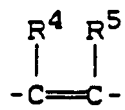

oder -C≡C- steht

worin

$R^4$ und $R^5$ gleich oder verschieden sind und Wasserstoff oder Alkyl mit bis zu 6 Kohlenstoffatomen bedeuten sowie deren Säureadditions-Salze.

Besonders bevorzugt sind die erfindungsgemäßen Verbindungen der allgemeinen Formel (I) in welcher

$R^1$

- für Wasserstoff oder
- für Alkyl mit bis zu 6 Kohlenstoffatomen oder
- für Alkylcarbonyl mit bis zu 4 Kohlenstoffatomen
- steht,

$R^2$

- für Cyano oder für die Gruppe

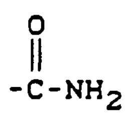

steht,

$R^3$

- für Phenyl steht, das gegebenenfalls bis zu 2-fach gleich oder verschieden substituiert ist durch Fluor, Chlor, Brom, Methyl, Ethyl, Isopropyl, tert.-Butyl, Methoxy, Methylthio, Trifluormethyl, Trifluormethoxy, Trifluormethylthio oder durch Phenyl oder Phenoxy, die ihrerseits durch Fluor, Chlor, Methyl oder Ethyl substituiert sein können,

X

- für ein Stickstoffatom oder für die CH-Gruppe steht,

Y

- für eine Bindung oder
- für eine Gruppe der Formel

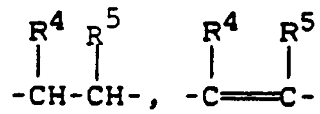

oder -C≡C- steht

worin

$R^4$ und $R^5$ gleich oder verschieden sind und Wasserstoff, Methyl oder Ethyl bedeuten,

sowie deren Säureadditions-Salze.

Bevorzugte erfindungsgemäße Verbindungen sind auch pharmakologisch verträgliche Additionsprodukte aus Säuren und den Verbindungen der allgemeinen Formel (I).

Zu den Säuren, die addiert werden können, gehören vorzugsweise Halogenwasserstoffsäuren, wie z. B. die Chlorwasserstoffsäure und die Bromwasserstoffsäure. insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z. B. p-Toluolsulfonsäure, 1,5-Naphthalindisulfonsäure oder Camphersulfonsäure.

Die erfindungsgemäßen Verbindungen der allgemeinen Formel (I) und deren Säureadditions-Salze zeigen gute antimikrobielle, insbesondere gute antimykotische Eigenschaften.

Die erfindungsgemäßen Stoffe enthalten ein asymmetrisch substituiertes Kohlenstoffatom. Sie können daher in Form von optischen Isomeren auftreten. Die vorliegende Erfindung betrifft sowohl die einzelnen Isomere als auch deren Gemische.

Folgende Beispiele der allgemeinen Formel (I) seien neben den bei den Herstellungsbeispielen genannten Verbindungen aufgeführt.

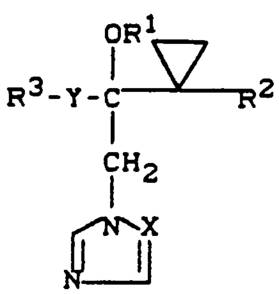

| $R^1$ | $R^2$ | $R^3$ | Y | X | Smp. |
|---|---|---|---|---|---|
| H | CN | 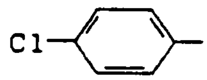 | $CH_2-CH_2$ | N | 156-7° C (A) |
| H | CN | 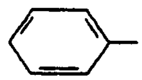 | CH=CH | N | (B) |
| H | CN | 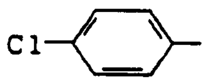 | CH=CH | N | (C) |
| H | CN | 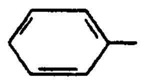 | C≡C | N | 110° C (D) |

(A) $^1$H-NMR ($CDCl_3$, δ-Werte in ppm):

0,45b (m, 1H), 0,73 (m, 1H), 1,0-1,2 (m, 2H), 1,85-2,2 (m, 2H), 2,7-3,0 (m, 2H), 4.25 (s, OH), 4,38 (d, 1H), 4,55 (d, 1H), 7,15 (dd, 2H), 7,25 (dd, 2H), 8,0 (s, 1H), 8,25 (s, 1H).

(D) Schmelzpunkt 110° C,
$^1$H-NMR ($CDCl_3$, δ-Werte in ppm):
1,2-1,4 (m, 4H), 4,6-4,75 (m, 2H), 5,5 (Br, s, OH), 7,25-7,4 (m, 5H), 7,95 (s, 1H), 8,3 (s, 1H).

Die Verbindungen der allgemeinen Formel (I)

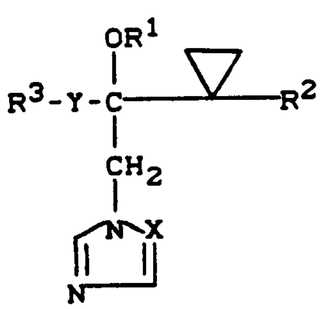
(I)

in welcher
$R^1$, $R^2$, $R^3$, X und Y die oben angegebene Bedeutung haben können stufenweise hergestellt werden, indem man

[A] Verbindungen der allgemeinen Formel (II)

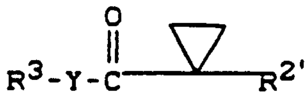 (II)

in welcher

$R^3$ und Y die oben angegebene Bedeutung haben und $R^{2'}$ für Cyano steht

mit Sulfonium- oder Sulfoxoniumsalzen der Formel (III)

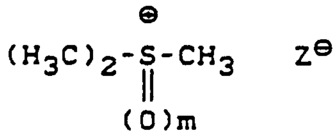 (III)

in welcher

Z - für Halogen steht und

m - eine Zahl 0 oder 1 bedeutet

in einem inerten Lösungsmittel, vorzugsweise Dimethylsulfoxid oder in Gemischen von Dimethylsulfoxid mit anderen inerten Lösemitteln, beispielsweise mit Tetrahydrofuran in Gegenwart einer Base, beispielsweise Dimsyl-Natrium, Natriumhydrid, Natriummethanolat, Natriumamid oder K-tert.-butanolat, bei Temperaturen von - 10 °C bis 100 °C umsetzt und die dabei entstehenden Oxirane der allgemeinen Formel (IV)

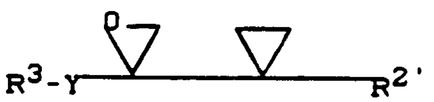 (IV)

in welcher

$R^3$ und Y die oben angegebene Bedeutung haben und $R^{2'}$ für Cyano steht mit Azolen der Formel (V)

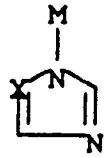 (V)

in welcher

X die oben angegebene Bedeutung hat und

M für Wasserstoff oder für ein Alkali- oder Erdalkalimetallatom steht gegebenenfalls in einem inerten Lösungsmittel und in Anwesenheit einer Base zu Verbindungen der Formel (I a)

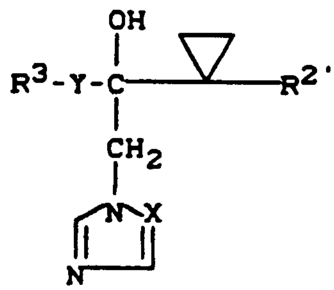

(Ia)

in welcher
$R^{2'}$, $R^3$, Y und X die oben angegebene Bedeutung haben umsetzt,
oder indem man
[B] Verbindungen der allgemeinen Formel (I a)

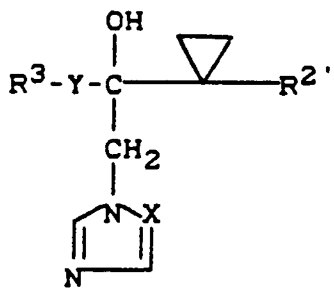

(I a)

in welcher
$R^3$, X und Y die oben angegebene Bedeutung haben und
$R^{2'}$ - für Cyano steht
unter den Bedingungen einer Phasentransferreaktion in einem Zweiphasensystem, bestehend aus einer wäßrigen Losung eines Alkalihydroxids, vorzugsweise Natrium- oder Kaliumhydroxid und einem inerten organischen Lösungsmittel, vorzugsweise Benzol, Toluol, Chloroform oder Dichlormethan unter Zugabe von Wasserstoffperoxid und einem Katalysator, beispielsweise Tetrabutylammoniumchlorid, Benzyltriethylammoniumhydroxid oder Tetrabutylammoniumhydrogensulfat, bei Temperaturen von -20 °C bis 60 °C partiell zu Verbindungen der allgemeinen Formel (I), in welcher $R^2$ für die Gruppe der Formel $-CO-NH_2$ steht, hydrolysiert,
oder indem man
[C] Azolylmethyl-cyclopropyl-carbinol-Derivate der Formel (I b)

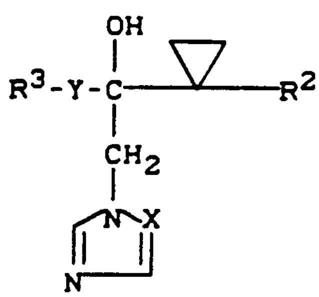

(I b)

in welcher
$R^2$, $R^3$, X und Y die oben angegebene Bedeutung haben,
mit starken Basen, wie beispielsweise Alkalimetallamiden oder - hydriden in Gegenwart eines Verdünnungsmittels, wie beispielsweise Dioxan, bei Temperaturen von 20 ° C bis 100 °C umsetzt und die dabei

entstehenden Alkoholate der allgemeinen Formel (I c)

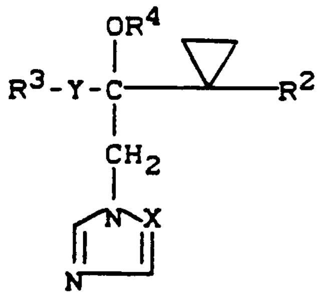

(I c)

in welcher
$R^2$, $R^3$, X und Y die oben angegebene Bedeutung haben und
$R^4$ - für einen kationischen Rest einer Base steht
mit Verbindungen der Formel (VI)

$R^5$-W (VI)

in welcher
$R^5$
- für Alkyl mit bis zu 10 Kohlenstoffatomen, oder Acyl mit bis zu 8 Kohlenstoffatomen steht
W
- für Halogen steht
in Gegenwart eines Verdünnungsmittels, beispielsweise Dioxan, bei Temperaturen von 20 °C bis 100 °C umsetzt
und
gegebenenfalls anschließend an die so erhaltenen Verbindungen der allgemeinen Formel (I) in üblicher Weise eine Säure oder ein Metallsalz addiert.

Es wurde außerdem ein neues Verfahren zur Herstellung der Verbindungen der allgemeinen Formel (II), in welcher $R^{2'}$, $R^3$ und Y die oben angegebene Bedeutung haben gefunden, indem man N-Methyl-N-Methoxyamide der Formel (VII)

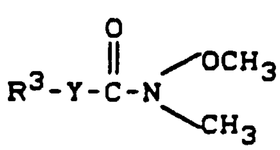

(VII)

in welcher
$R^3$ und Y die oben angegebene Bedeutung haben
mit dem Lithiumsalz von 1-Cyano-Cyclopropan der Formel

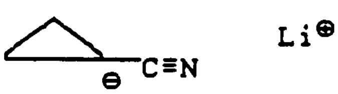

(VIII)

in einem inerten Lösungsmittel, wie beispielsweise Tetrahydrofuran oder Diethylether in einem Temperaturbereich von -100 °C bis +20 °C umsetzt.

Die Verbindungen der Formeln (VII) und (VIII) sind bekannt (S. Weinreb, Tetrahedron Lett. 22 (1981), 3815, H.W. Pinnick et al., J. Org. Chem. 45 (1980) 4506).

Die Verbindungen der Formel (III) sind ebenfalls bekannt (C.Ferri, Reaktionen der organischen Chemie, Thieme-Verlag, 1978).

Die bei der Durchführung des erfindungsgemäßen Verfahrens [A] als Reaktionskomponente benötigten Azole der Formel (V) sind allgemein bekannte Verbindungen der organischen Chemie.

Die Halogenverbindungen der Formel (VI) sind bekannt oder lassen sich nach im Prinzip bekannten Methoden herstellen.

Die Durchführung des Verfahrens [A] kann sowohl in einer Einstufenreaktion als auch unter Isolierung der einzelnen Zwischenprodukte erfolgen. Die Umsetzung erfolgt in einem Temperaturbereich von -20 °C bis 200 °C, vorzugsweise bei -10 °C bis 100 °C, bei erhöhtem oder normalem Druck.

Als Lösungsmittel für die Verfahren [A] und [C] eignen sich inerte Lösemittel wie beispielsweise N,N-Dimethylformamid, N,N-Dimethylacetamid, Dimethylsulfoxid, Sulfolan, N-Methylpyrrolidon-2, Dioxan, Tetrahydrofuran, Acetonitril, Methylenchlorid oder ein aromatischer Kohlenwasserstoff wie Benzol, Chlorbenzol, Nitrobenzol, Toluol oder Xylol. Es können auch Gemische der beispielhaft genannten Lösungsmittel verwendet werden.

Als Basen bei dem Verfahren [A] eignen sich beispielsweise Alkalimetall- oder Erdalkalimetall-carbonate, hydrogen-carbonate, -hydroxide, -alkoholate oder -hydride wie z.B. Natriumcarbonat, Natriumhydrogencarbonat, Kaliumcarbonat, Natriumhydroxid, Natriummethylat oder Natriumhydrid, oder organische Basen, z.B. tertiäre Amine wie Triethylamin, Tributylamin, Ethylmorpholin oder Pyridin, Dimethylaminopyridin, Chinolin oder 1,5-Diazabicyclo [5,4,0] undec-5-en(1,8,7) (DBU).

Die Basen werden bei den Verfahren [A] und [C] in äquimolaren Mengen oder mit einem 2-3-fachen molaren Überschuß. bezogen auf die anderen Reaktionskomponenten eingesetzt. Bevorzugt ist der Einsatz mit 2-fach molarem Überschuß

Als Lösungsmittel für das Verfahren [B] eignen sich aromatische Kohlenwasserstoffe wie Benzol, Toluol oder aliphatische, chlorierte Kohlenwasserstoffe wie beispielsweise Dichlormethan oder Chloroform.

Als Basen können Alkalimetall- oder Erdalkalimetallhydroxide, Alkali- oder Erdalkalihydride oder Alkali- oder Erdalkalialkoholate, wie beispielsweise Natriumhydroxid, Kaliumhydroxid, Natriumhydrid, Natriummethanolat oder Natriumethanolat, eingesetzt werden.

Die Basen werden in einem 1-3-fachen molaren Überschuß, bezogen auf die übrigen Reaktionskomponenten, eingesetzt.

Die bei dem erfindungsgemäßen Verfahren [C] als Ausgangsstoffe benötigten Azolylmethylcyclopropyl-Derivate der Formel (Ib) sind erfindungsgemäße Verbindungen. Ihre Überführung in die entsprechenden Alkoholate erfolgt in allgemein bekannter Weise, indem man sie mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^4$ in den Verbindungen der Formel (Ic) vorzugsweise für ein Alkalimetallkation, wie ein Natrium- oder Kaliumkation, oder für ein quarternäres Ammonium- oder Phosphoniumkation.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel, z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise oral oder lokal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,1 bis etwa 200, vorzugsweise 1 bis 50 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 1 bis etwa 200, vorzugsweise 2 bis 100 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

1-(1-Cyanocyclopropyl)-1-phenyl-2-(1,2,4-triazol-1-yl)ethanol

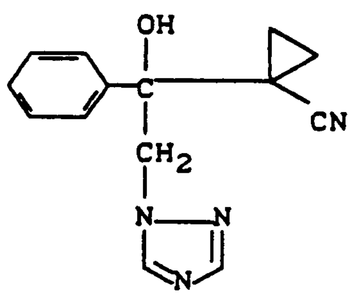

(I-1)

660 mg (0,022 mol) 80%iges Natriumhydrid (in Öl) werden unter Argon in 30 ml abs. DMSO suspendiert. Man gibt bei RT 4,8 g (0.022 mol) Trimethylsulfoxoniumjodid (EGA) hinzu und rührt bis zum Ende der Wasserstoffentwicklung. Dann werden rasch 3,4 g (20 mmol) 1-Benzoyl-1-cyanocyclopropan in 10 ml abs. DMSO zugegeben. Man rührt 2 h bei 40 °C und gibt dann 2 g (30 mmol) 1,2,4-Triazol subl. und 908 mg (10 mmol) 1,2,4- Triazol-Na-Salz (EGA) hinzu. Man rührt 2 h bei 50 ° C und 4 h bei 80 °C, kühlt, rührt in Eiswasser ein und extrahiert mit Chloroform. Die organische Phase wird mit 2 n NaOH gewaschen, getrocknet ($Na_2SO_4$) und eingeengt. Man chromatograhiert an Kieselgel mit Essigester und kristallisiert aus Dichlormethan/Petrolether um. Man erhält 2 g (40 %. d. Th.) 1-(1-Cyanocyclopropyl)-1-phenyl-2-(1,2,4-triazol-1-yl)ethanol mit dem Schmelzpunkt 103-105 °C als farblose Kristalle,
$^1$H-NMR ($CDCl_3$):
$\delta$ = 0.9-1.1(m,4H), 4.7(d,1H),4.9 (d,1H), 5.13 (s,1H), 7.3-7.5 (m,3H), 7,65(m,2H), 7.95 (s, 1H, 8,25(s,1H) ppm.

Beispiel 2

1-(1-Cyanocyclopropyl)-1-(4-fluorphenyl)-(1,2,4-triazol-1-yl )-ethanol

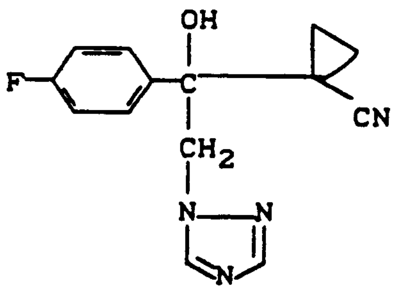

(I-2)

Zur Suspension aus 0,33 g (11 mmol) Natriumhydrid (80 % in Öl) in 20 ml DMSO p.a. gibt man bei 20 °C portionsweise 2,42 g (11 mmol) Trimethylsulfoxoniumjodid (EGA). Man rührt bis zum Ende der Wasserstoffentwicklung und tropft 2 g (10 mmol) (4-Fluoro)-1-benzoyl-1-cyanocyclopropan, gelöst in 5 ml abs. DMSO schnell zu. Man rührt 1 h bei RT und 1 h bei 40 °C, dann werden 1,04 g (15 mmol) 1,2,4-Triazol (sublimiert) und 0,46 g (5 mmol) 1,2,4-Triazol-Na-Salz in DMSO zugegeben. Man rührt 2 h bei 50 °C und 4 h bei 80 °C, gießt in Eiswasser und extrahiert mit Dichlormethan. Die organische Phase wird mit 2 N Natronlauge gewaschen, über $Na_2SO_4$ getrocknet und eingeengt. Man chromatographiert an Kieselgel und kristallisiert aus Diethylether. Man erhält so 1,2 g (44 % d. Th.) farblose Kristalle 1-(1-Cyanocyclopropyl)-1-(4-fluorphenyl)-2-(1,2,4-triazol-1-yl)-ethanol mit dem Schmelzpunkt 121-122 °C,
$^1$H-NMR ($CDCl_3$): $\delta$ = 0.95-1.1(m,4H), 4.7(d,1H), 4.9(d,1H), 5.25(s,1H), 7.10 (t,2H), 7.65(dd,2H), 7.9(s, 1H), 8.28(s,1H) ppm.

Beispiel 3

1-(1-Cyanocyclopropyl-)-1-(4-chlorphenyl)-2-(1,2,4-triazol-1-yl)-ethanol

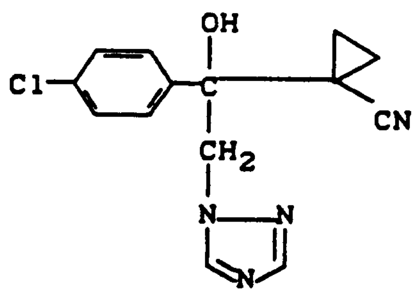

(I-3)

750 mg (0.025 mol) Natriumhydrid (80 %ig in Öl) werden unter Argon in 30 ml abs. DMSO suspendiert. Man gibt bei RT 5,5 g (25 mmol) Trimethylsulfoxoniumjodid (EGA) hinzu. Nach Ende der $H_2$-Entwicklung werden 4,6 g (25 mmol) des Ketons II-3 (Tabelle 2) zugegeben (in 30 ml abs. DMSO). Man rührt 2 h bei 40 °C und gibt dann 2,3 g (34 mmol) 1,2,4-Triazol (EGA) und 1,03 g (11 mmol) 1,2,4-Triazol-Natriumsalz (EGA) zu. Man rührt 2 h bei 40 °C und 4 h bei 80 °C, kühlt, rührt in Eiswasser ein und extrahiert mit Essigsäureethylester. Die organische Phase wird mit 2 n Natronlauge gewaschen, mit $Na_2SO_4$ getrocknet und eingedampft. Man chromatographiert an Kieselgel mit Essigester und kristallisiert aus Dichlormethan/Petrolether um. Man erhält 1.6 g (22 % d.Th.) 1-(1-Cyanocyclopropyl-)1-(4-chlorphenyl)-2-(1,2,4-triazol-1-yl)-ethanol als farblose Kristalle vom Schmelzpunkt 107 °C, $^1$H-NMR ($CDCl_3$):
$\delta$ = 0.9-1.1(m,4H), 4.70(d,1H) 4,90(d,1H), 5.24(s.1H), 7.4 (dd,2H), 7.6(dd,2H), 7.95 (s,1H), 8.28(s,1H) ppm.

Beispiel 4

1-(1-Cyanocyclopropyl)-1-(2,4-difluorphenyl)-2-(1,2,4-triazol-1-yl)ethanol

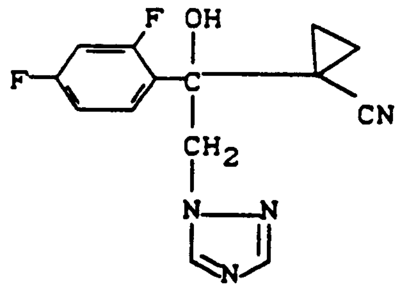

(I-4)

990 mg (0.033 mol) Natriumhydrid (80%ig in Öl) werden in 60 ml abs. DMSO suspendiert. Bei RT werden 7,2 g (0.033 mol) Trimethylsulfoxoniumjodid (Aldrich) zugegeben. Nach Ende der $H_2$-Entwicklung werden rasch 6,2 g (0.03 mol) des Ketons II-12 (Tabelle 2) in 30 ml abs. DMSO zugegeben. Man rührt 1 h bei RT und 1 h bei 40 °C, gibt 3 g (45 mmol) 1,2,4-Triazol subl. und 1,36 g (15 mmol) 1,2,4-Triazol-Natriumsalz (EGA) zu, rührt 2 h bei 50 °C und 5 h bei 80 °C. Man kühlt, rührt in Wasser ein und extrahiert mit Essigester. Die organische Phase wird mit 2 n NaOH gewaschen, über $Na_2SO_4$ getrocknet und eingedampft. Man chromatograhiert an Kieselgel mit Essigester und kristallisiert aus Essigester/Petrolether. Man erhält 3,6 g (41 % d.Th.) 1-(1-Cyanocyclopropyl)-1-(2,4-difluorphenyl)-2-(1,2,4-triazol-1-yl)ethanol als farbl. Kristalle mit dem Schmelzpunkt 103-105 °C, $^1$H-NMR ($CDCl_3$):

$\delta$ = 1.1-1.25(m,4H), 4.79(d,1H), 5.28 d,1H), 5.55(s.1H), 6.80(ddd,1H), 6.86(ddd,1H), 7.60(m,1H) 7.85 (s,1H) 8.20(s,1H) ppm.

Beispiel 5

1-(1-cyanocyclopropyl)-1-(4-fluorphenyl)-2-imidazol-1-yl)-ethanol

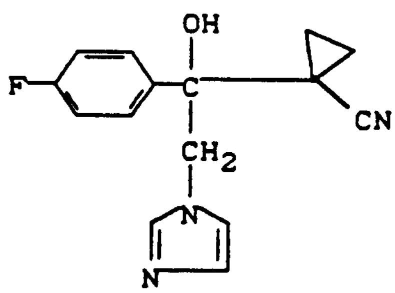

(I-5)

Unter Stickstoff-Atmosphäre werden 4 g (59 mmol) Imidazol und 2,1 g (10 mmol) 2-(1-Cyanocyclopropyl)-2-(4-fluorphenyl)-oxiran (IV-2, Beispiel 33) in 20 ml abs. Acetonitril 8 h unter Rückfluß erhitzt. Nach dem Abdampfen des Lösemittels im Vakuum wird der Rückstand in Essigsäureethylester aufgenommen. Man wäscht mit Wasser, trocknet über $Na_2SO_4$ und dampft das Lösemittel im Vakuum ab. Chromatographische Reinigung an Kieselgel (Laufm.: Dichlormethan mit 2 % Ethanol) ergibt 1.1 g (39 % d.Th.) 1-(1-Cyanocyclopropyl)-1-(4-fluorphenyl)-2(imidazol-1-yl)-ethanol mit dem Schmelzpunkt 173 °C (Zers.), $^1$H-NMR ($CDCl_3$):

$\delta$ = 0,85-1.24(m,4H), 4.5 (d,1H), 4.65 (d, 1H), 6.68(s,1 H) ,6.95 (s,1H), 7.10 (t,2H), 7.53 (s,1H), 7.65 (dd,2H) ppm.

Beispiel 6

2-(1-Cyanocyclopropyl)-4-phenyl-1-(1,2,4-triazol-1-yl)-butan-2-ol

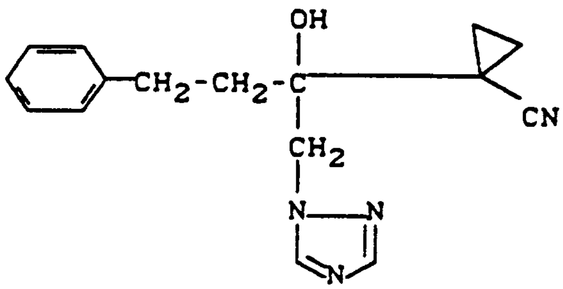

(I-6)

0,8 g (12 mmol)1,2,4-Triazol, 0,1 g (1 mmol) Kaliumtert. butanolat und 0,8 g (4 mmol) 2-(1-Cyanocyclopropyl)-2-(2-phenylethyl)-oxiran (IV-16, Beispiel 34) werden in 10 ml abs. DMF 6 h auf 80 °C erhitzt. Man zieht das Lösemittel im Vakuum ab, nimmt den Rückstand in Essigsäureethylester auf und wäscht mit Wasser. Nach dem Trocknen über $Na_2SO_4$ wird das Lösemittel im Vakuum abgedampft, der Rückstand an Kieselgel chromatographiert ($CH_2Cl_2$ mit 2 % Ethanol). Man erhält 0,3 g (28 % d.Th.) an 2-(1-Cyanocyclopropyl)-4-phenyl-1-(1,2,4-triazol-1-yl)-butan-2-ol in Form eines farblosen Öles, $^1$H-NMR ($CDCl_3$):
$\delta$ = 0.42-0,5(m,1H), 0.68-0.78(m,1H), 1.03-1.22(m,2H), 1.93-2.05(m,1H), 2.15(m,1H), 2.8(m,1H), 2.95(m,1H), 4.27(s,1H), 4.41(d,1H), 4.57(d,1H), 7.2-7.34(m,5H), 8.02(s,1H), 8.27(s,1H) ppm.

Beispiel 7:

1-(1-Carboxamidocyclopropyl)-1-(4-fluorphenyl)-2-(1,2,4-triazol-1-yl)-ethanol

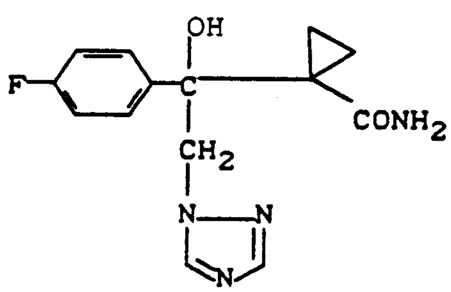

(I-7)

3,0 g (0.011 mol) des Nitrils I-2 (Beispiel 2) werden in 30 ml Chloroform gelöst. Man kühlt auf 0 °C und gibt 10,3 ml (100 mmol) 30 %ige $H_2O_2$-Lösung, 1 g Tetrabutylammoniumhydrogensulfat sowie 8.4 ml 20 %ige wässrige NaOH hinzu. Während 24 Stunden werden weitere 20 ml 30 %ige $H_2O_2$-Lösung unter Rühren hinzugefügt. Man verdünnt mit Dichlormethan, trennt die organische Phase ab, trocknet mit $Na_2SO_4$ und dampft ein. Man kristallisiert aus Methanol und erhält 1,9 g (60 % d.Th.) 1-(1-Carboxamidocyclopropyl)-1-(4-fluorphenyl)-2-(1,2,4-triazol-1-yl)-ethanol mit dem Schmelzpunkt 209 °C, $^1$H-NMR ($CDCl_3$ + DMSO-$d_6$):
$\delta$ = 0.8-1.1 (m,4H), 4.65 (d,1H), 4.85 (d,1H) 6.3(s,1H), 6.65(br.s,1H) 6.95(dd, 2H), 7.1(br.s,1H), 7.45(dd,2H), 7.7 (s,1H), 8.2(s,1H) ppm.

**Tabelle 1**

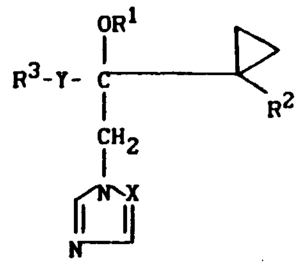

| Bsp.Nr. | Verb.Nr. | $R^3$ | $R^1$ | $R^2$ | X | Y | Schmelzpunkt (°C) | Spektroskop.Daten ($^1$H-NMR, $CDCl_3$, δ-Werte in ppm) |
|---|---|---|---|---|---|---|---|---|
| 8 | I-8 | 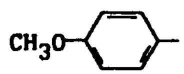 | H | CN | N | - | 88-90 | 0.9-1.05(m,4H),3.8(s,3H), 4.7(d,1H),4.9(d,1H),4.95 (s,1H), 6.90(m,2H),7.55(m, 2H),7.95(s,1H),8.23(s,1H) |
| 9 | I-9 | 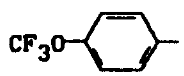 | H | CN | N | - | 57-59 | 0.9-1.1(m,4H),4.7(d,1H), 4.95(d,1H),5.3(s,1H), 7.25 (dd,2H),7.70(dd,2H), 7.95(s,1H), 8.30(s,1H) |
| 10 | I-10 | 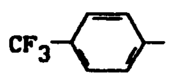 | H | CN | N | - | amorph | 0.9-1.1(m,4H),4.7(d,1H), 4.95(d,1H),5.3(s,1H),7.68 (d,2H),7.80(d,2H),8.0(s, 1H),8.3(s,1H) |

| Bsp.Nr. | Verb.Nr. | $R^3$ | $R^1$ | $R^2$ | X | Y | Schmelzpunkt (°C) | Spektroskop.Daten ($^1H$-NMR, $CDCl_3$, δ-Werte in ppm) |
|---|---|---|---|---|---|---|---|---|
| 11 | I-11 | 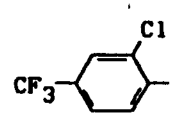 | H | CN | N | - | 160-162 | 1,05(m,1H),1.19(m,2H),1,35 (m,1H),4.8(d,1H),5.6 (s,1H), 5,68(d,1H),7.53 (dd,1H), 7.65(s,1H)7,93 (s,1H),7,95 (dd,1H),8.20 (s,1H) |
| 12 | I-12 | 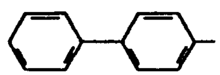 | H | CN | N | - | 128-130 | 0.9-1.1(m,4H),4.75(d,1H), 4.95(d,1H),5.2(s,1H), 7.3-7.8(m,9H),7.95(s,1H), 8.3 (s,1H) |
| 13 | I-13 | 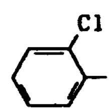 | H | CN | N | - | 131-133 | 1,0-1.2(m,3H),1.35(m,1H), 4.75(d,1H),5.4(s,1H),5.6 (d,1H),7.25(m.2H),7.35 (m,1H),7.75(m,1H),7.85 (s,1H),8.20(s,1H) |
| 14 | I-14 | 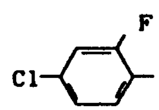 | H | CN | N | - | 120-122 | 1.1-1.3(m,4H),4.75 (d,1H),5.3 (d,1H),5.55 (s,1H),7.1(dd,1H),7.15 (dd,1H),7.55(t,1H), 7.85 (s,1H),8.20(s,1H) |

| Bsp.Nr. | Verb.Nr. | $R^3$ | $R^1$ | $R^2$ | X | Y | Schmelzpunkt (°C) | Spektroskop.Daten ($^1$H-NMR, $CDCl_3$, δ-Werte in ppm) |
|---|---|---|---|---|---|---|---|---|
| 15 | I-15 | 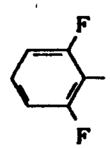 | H | CN | N | - | 99-101 | 1.15-1.3(m,4H),4.7(d,1H), 5.4(d,1H), 5.6(s,1H), 6.9 (dd,2H), 7.25-7.35(m,1H), 7.9(s,1H), 8.3(s,1H) |
| 16 | I-16 | 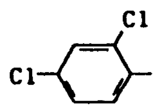 | H | CN | N | - | 125 | 1.0-1.2(m,3H),1.35(m,1H), 4.7(d,1H),5.5(s,1H),5.65 (d,1H), 7.23(dd,1H), 7.4 (d,1H), 7.7(d,1H),7.9(s,1H), 8.2(s,1H) |
| 17 | I-17 | 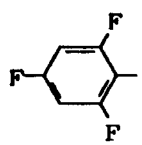 | H | CN | N | - | amorph | 1.15-1.35(m,4H), 4.65 (d,1H),5,35(s,1H), 5.65 (s,1H), 6.65(m,2H), 7.90(s,1H), 8.28(s,1H) |

Beispiel 18

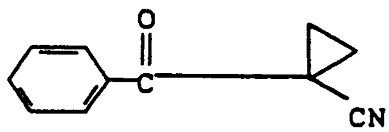

(II-1)

Verfahrensvariante A:

1 g (7 mmol) α-Cyanoacetophenon, 8 g (43 mmol)1,2-Dibromethan, 0,1 g (0,4 mmol) 18-Krone-6 und 4 g(69 mmol) Kaliumfluorid werden als heterogenes System in 10 ml Dichlormethan 18 h unter Rückfluß erhitzt. Nach dem Abkühlen wird filtriert, die organische Phase mit $H_2O$ gewaschen und nach dem Trocknen über $Na_2SO_4$ das Lösungsmittel im Vakuum abgedampft. Der Rückstand wird mit $CH_2Cl_2$ an Kieselgel chromatograhiert. Man erhält auf diese Weise 0.25 g (20 % d.Th.) 1-Benzoyl-1-Cyanocyclopropan als farbloses Öl,

$^1$H-NMR ($CDCl_3$): δ = 1.75 (m,2H), 1.87(m,2H), 7.5-7.7(m,3H),8.0(dd,2H) ppm.

Verfahrensvariante B:

Unter Argon werden 13.2 ml (0.096 mol) Diisopropylamin und 75 ml THF p.a. auf -78 ° C gekühlt und tropfenweise mit 58.6 ml (0,096 mol) 1.6 M n-Butyllithium in n-Hexan versetzt. Man rührt 30 Min. bei -78 ° C, gibt 7 ml (0.096 mol) Cyclopropylnitril (EGA) zu und rührt 1 h bei -78 ° C nach. Man tropft 13,6 g (0.0825 mol) N-Methoxy-N-methylbenzamid (S. Weinreb, THL 1981, 3815) hinzu (in 50 ml abs. THF) und rührt 4 h bei 0 ° C. Man gießt in Eiswasser, neutralisiert mit 5 %iger HCl. Man gibt Ether zu, extrahiert mit Dichlormethan, trocknet mit $Na_2SO_4$ und dampft ein. Man chromatograhiert an Kieselgel mit Dichlormethan und erhält 7.1 g (50 % d.Th.) 1-Benzoyl-1-Cyanocyclopropan als farbloses Öl.

Vorstufen

Beispiel 19

1-(4-Fluoro)benzoyl-1-cyanocyclopropan-1-on

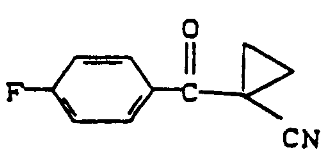

(II-2)

57,5 ml (0.41 mol) Diisopropylamin und 290 ml abs. THF werden unter Argon auf -78 ° C gekühlt. Man tropft 255 ml (0.41 mol) 1.6 M n-Butyllithium in Hexan zu und rührt 30 Min. bei -78 ° C. 30.1 ml (0.409 mol) Cyclopropylnitril werden langsam zugetropft, 1 h wird bei -78 ° C nachgerührt. Man tropft 65.1 g (0.355 mol) 4-Fluor-(N-methyl-N-methoxy)benzamid in 200 ml abs. THF zu und läßt langsam auf Raumtemperatur kommen. Man gießt auf 500 ml 5 % HCl/Eiswasser, stellt neutralen pH ein, versetzt mit gesättigter NaCl-Lösung und extrahiert mit Essigsäureethylester. Man trocknet über $Na_2SO_4$, dampft ein und kristallisiert aus Diethylether. Man erhalt 33.8 g (50.4 % d.Th.) farblose Kristalle vom Schmelzpunkt 60-62 ° C,

$^1$H-NMR ($CDCl_3$): δ = 1.75 (m,2H), 1 .86 (m2H), 7.2 (m,2H), 8.1 (m,2H) ppm.

**Tabelle 2**

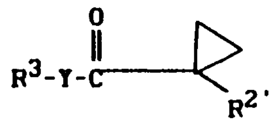

| Bsp.Nr. | Verb.Nr. | $R^3$ | $R^{2'}$ | Y | Schmelzpunkt (°C) | Spektroskop.Daten, ($^1$H-NMR, $CDCl_3$, δ-Werte in ppm) |
|---|---|---|---|---|---|---|
| 20 | II-3 | 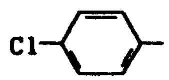 | CN | Bindung | 68-70° C | 1.75(m,2H), 1.86 (m,2H), 7.5 (dd,2H), 8.0(dd,2H) |
| 21 | II-4 | 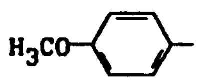 | CN | Bindung | Öl | 1.70(m,2H),1.80 (m,2H), 3.85(s,3H),7.0(dd,2H), 8.1 (dd,2H) |
| 22 | II-5 | 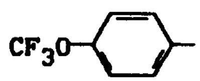 | CN | Bindung | Öl | 1.75 (m,2H) 1.87 (m,2H), 7.35(dd,2H),8.12(dd,2H) |
| 23 | II-6 | 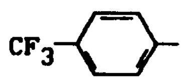 | CN | Bindung | Öl | 1.82 (m,2H), 1.93 (m,2H), 7.8 (d,2H), 8.15 (d,2H) |
| 24 | II-7 | 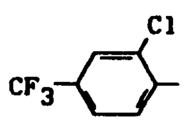 | CN | Bindung | amorph | 1.9 (m,2H), 2.02 (m,2H), 7.55 (d,1H), 7.65 (d,2H), 7.76 (s,1H) |
| 25 | II-8 | 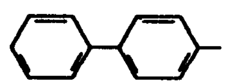 | CN | Bindung | 118° C | 1.75 (m,2H), 1.9 (m,2H), 7.45 (m,3H), 7.6 (dd,2H), 7.73(dd,2H),8.13(dd,2H) |

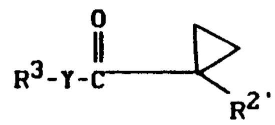

| Bsp.Nr. | Verb.Nr. | $R^3$ | $R^{2'}$ | Y | Schmelzpunkt (°C) | Spektroskop.Daten, ($^1$H-NMR, $CDCl_3$, δ-Werte in ppm) |
|---|---|---|---|---|---|---|
| 26 | II-9 | 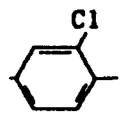 | CN | Bindung | Öl | 1.85(m,2H), 1.95 (m,2H), 7.3-7.6 (m,4H) |
| 27 | II-10 | 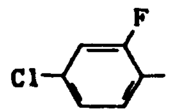 | CN | Bindung | | 1.8(m,2H), 1.95 (m,2H), 7.25(m,2H),7.55(dd,1H) |
| 28 | II-11 | 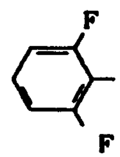 | CN | Bindung | | 1.85 (m,2H) 2.0 (m,2H), 7.03(dd,2H),7.5(m,1H) |
| 29 | II-12 | 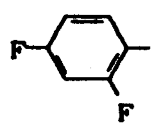 | CN | Bindung | Öl | 1.8 (m,2H), 1.95 (m,2H), 6.9-7.1 (m,2H),7.65(m,1H) |
| 30 | II-13 | 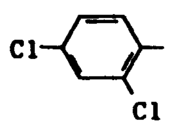 | CN | Bindung | | 1.85 (m,2H), 2.0 (m,2H), 7.4 (s,2H), 7.5 (s,1H) |

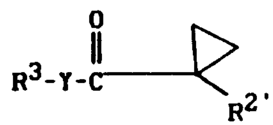

| Bsp.Nr. | Verb.Nr. | $R^3$ | $R^{2'}$ | Y | Schmelzpunkt (°C) | Spektroskop.Daten, ($^1H$-NMR, $CDCl_3$, δ-Werte in ppm) |
|---|---|---|---|---|---|---|
| 31 | II-14 | 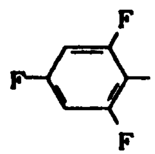 | CN | Bindung | | 1.85 (m,2H), 1.96(m,2H) 6.8 (m,2H) |
| 32 | II-16 | 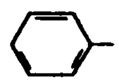 | CN | $CH_2$-$CH_2$ | | 1.6 (m,2H), 1.65(m,2H), 2.95 (t,2H), 3.25 (t,2H) 7.2-7.4 (m,5H) |

Beispiel 33

2-(1-Cyanocyclopropyl)-2-(4-fluorphenyl)-oxiran

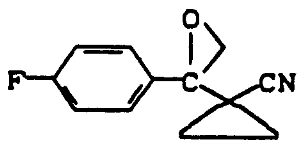

(IV-2)

Zu 6.6 g (0.22 mol) Natriumhydrid (80 %ig in Öl) und 34.2 g (0.16 mol) Trimethylsulfoxoniumiodid tropft man bei 10 °C langsam 130 ml abs. DMSO. Es wird eine Stunde bei 20 °C gerührt und anschließend 26.3 g (0.14 mol) 1-Cyanocyclopropyl-4-fluorphenylketon (Verbindung II-2, Beispiel 19, gelöst in 65 ml abs. DMSO) zugetropft. Man rührt 14 h bei 20 °C. Die Lösung wird auf Wasser gegossen und mit Essigsäureethylester extrahiert. Die vereinigten organischen Phasen werden mit $H_2O$ gewaschen, über $Na_2SO_4$ getrocknet und das Lösemittel im Vakuum abgedampft. Man chromatograhiert an Kieselgel und erhält nach Abdampfen des Lösemittels 15 g (54 % d.Th.) an 2-(1-Cyanocyclopropyl)-2-(4-fluorphenyl)-oxiran in Form eines farblosen Öles, $^1$H-NMR ($CDCl_3$):
$\delta$ = 1.03-1.40 (m,4H), 2.95 (d,1H), 3.09 (d,1H), 7.07 (t,2H), 7.49 (dd,2H) ppm.

Beispiel 34

2-(1-Cyanocyclopropyl)-2-(2-phenylethyl)-oxiran

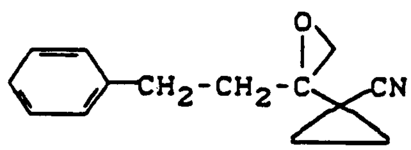

(IV-16)

Zu 2 g (10 mmol) Trimethylsulfoniumjodid und 0,2 g (7 mmol) Natriumhydrid (80 %ig in Öl) tropft man bei 10 °C 10 ml abs. DMSO. Es wird 1 h bei 20 °C nachgerührt. Hiernach tropft man eine Losung aus 1 g (5 mmol) 1-Cyanocyclopropyl-2-phenylethylketon (Verb. II-16, Beispiel 32) in 5 ml abs. DMSO zu und läßt 4 h bei 20 °C reagieren. Die Losung wird auf Wasser gegossen, mit Essigsäureethylester extrahiert, die organische Phase mit $Na_2SO_4$ getrocknet und das Lösemittel im Vakuum abgedampft. Man erhält 0.8 g (75 % d. Th.) in Form eines farblosen Öles,
$^1$H-NMR ($CDCl_3$):
$\delta$ = 0.8-1.4 (m,4H) 2.03-3.20 (m, 1H) 2.24-2.42 (m, 1H), 2.57-2.9 (m, 4H) 7.14-7.33 (m, 5H) ppm.

**Tabelle 3**

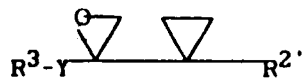

| Bsp.Nr. | Verb.Nr. | $R^3$ | $R^{2'}$ | Y | Schmelzpunkt (°C) | Spektroskop.Daten ($^1$H-NMR, $CDCl_3$, δ-Werte in ppm) |
|---|---|---|---|---|---|---|
| 35 | IV-1 | 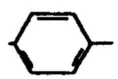 | CN | Bindung | Öl | |
| 36 | IV-3 | 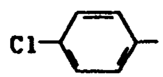 | CN | Bindung | Öl | |
| 37 | IV-4 | 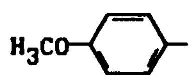 | CN | Bindung | Öl | |
| 38 | IV-5 | 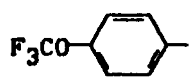 | CN | Bindung | Öl | |
| 39 | IV-6 | 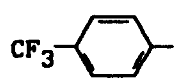 | CN | Bindung | Öl | 0.9 -1.40(m,4H), 3.0 (d,1H), 3.1 (d,1H), 7.1-7.5 (m,4H) |
| 40 | IV-7 | 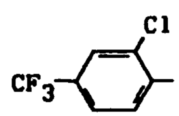 | CN | Bindung | Öl | |

| Bsp.Nr. | Verb.Nr. | $R^3$ | $R^{2'}$ | Y | Schmelzpunkt (°C) | Spektroskop.Daten ($^1H$-NMR, $CDCl_3$, δ-Werte in ppm) |
|---|---|---|---|---|---|---|
| 41 | IV-8 | 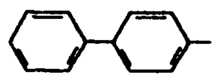 | CN | Bindung | Öl | |
| 42 | IV-9 | 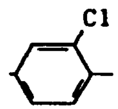 | CN | Bindung | Öl | |
| 43 | IV-10 | 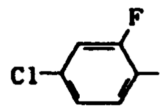 | CN | Bindung | Öl | |
| 44 | IV-11 | 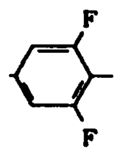 | CN | Bindung | Öl | |
| 45 | IV-12 | 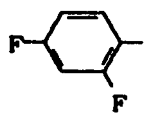 | CN | Bindung | Öl | |
| 46 | IV-13 | 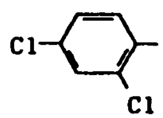 | CN | Bindung | Öl | 0.9-1.3(m,4H),2.90(d,1H), 3.26(d,1H),7.2-7.4(m,3H) |

| Bsp.Nr. | Verb.Nr. | $R^3$ | $R^{2'}$ | Y | Schmelzpunkt (°C) |
|---|---|---|---|---|---|
| 47 | IV-14 | 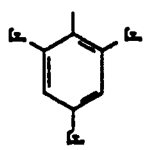 | CN | Bindung | Öl |
| 48 | IV-18 | 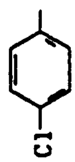 | CN | $CH_2-CH_2$ | Öl |
| 49 | IV-19 | 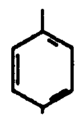 | CN | CH=CH | Öl |
| 50 | IV-20 | 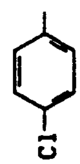 | CN | CH=CH | Öl |
| 51 | IV-21 | 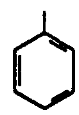 | CN | C≡C | Öl |

Verwendungsbeispiele

In den nachfolgenden Verwendungsbeispielen werden die nachstehend angegebenen Substanzen als Vergleichsverbindungen eingesetzt: A, B (bekannt aus der EP-OS 44 605) sowie C, D (bekannt aus der EP-OS 180 850).

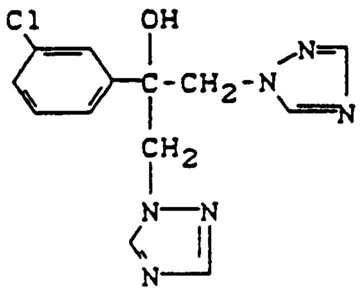

A

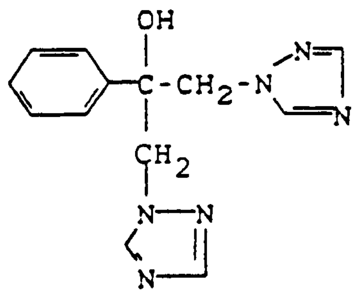

B

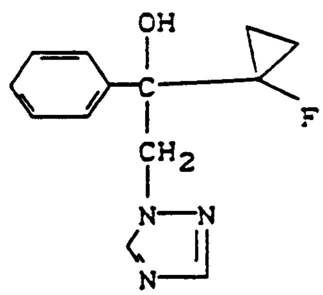

C

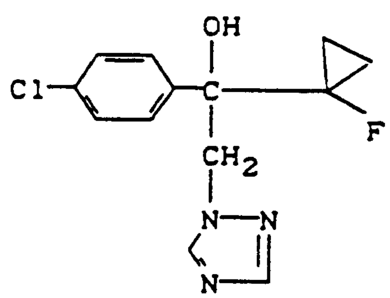

D

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^4$ bis $10^5$ Keimen/ml Substrat durchgeführt. Als Nährmedium dienten

a) für Dermatophyten und Schimmelpilze:

Sabourauds's milieu d'epreuve

b) für Hefen:
Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigen z. B. die erfindungsgemäßen Verbindungen (I-3), (I-5), (I-14) und (I-16) eine bessere Wirksamkeit, insbesondere gegenüber Candida albicans, als die aus dem Stand der Technik bekannten Verbindungen (A) und (B).

Tabelle A:

Antimykotische in-vitro Wirksamkeit

| Wirkstoff | MHK-Werte in µg/ml Nährmedium Trichophyton mentagr. | Microsporum canis | Candida albicans | Torulopsis glabrata | Aspergillus fumigatus |
|---|---|---|---|---|---|
| (A) (bekannt) | 32 | - | > 64 | > 64 | > 64 |
| (B) (bekannt) | 64 | - | 64 | > 64 | > 64 |
| Verbindungen gemäß Herstellungsbeispielen: | | | | | |
| I-3 | 2 | 4 | 2 | 8 | 4 |
| I-5 | < 1 | 4 | 8 | 8 | 4 |
| I-14 | < 1 | 4 | 2 | 16 | 4 |
| I-16 | < 1 | < 1 | 16 | 32 | 2 |

Beispiel B

Antimykotische in-vivo-Wirksamkeit (oral) bei Mäusecandidose

Versuchsbeschreibung:

Mäuse vom Typ SPF-$CF_1$ wurden intravenös mit 1 - 2 x $10^6$ logarithmisch wachsenden Candida-Zellen, die in physiologischer Kochsalzlösung suspendiert wurden, infiziert. Eine Stunde vor und sieben Stunden nach der Infektion wurden die Tiere mit jeweils 10-100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5 %.

In diesem Test zeigen z. B. die erfindungsgemäßen Verbindungen (I-2), (I-3), (I-4,) (I-7), (I-9), (I-11), (I-13), (I-14), (I-15) und (I-16) gute bis sehr gute Wirkung, d. h. > 80 % Überlebende am 6.Tag p.i. und sind damit besser als die aus dem Stand der Technik bekannten Verbindungen (C) und (D).

Zeichenerklärung:

| | | |
|---|---|---|
| + + + + + | = sehr gute Wirkung | = 90% Überlebende am 6.Tag p.i. |
| + + + + | = gute Wirkung | = 80% Überlebende am 6.Tag p.i. |
| + + + | = Wirkung | = 60% Überlebende am 6.Tag p.i. |
| + + | = schwache Wirkung | = 40% Überlebende am 6.Tag p.i. |
| + | = Spur Wirkung | = unter 40% Überlebende am 6.Tag p.i. |
| k.W. | | = kein Unterschied zur unbehandelten Infektionaskontrolle |

Tabelle B

| Antimykotische in-vivo-Wirksamkeit (oral) bei Mäusecandidose | |
|---|---|
| Wirkstoff | Wirkung |
| (C) (bekannt) | k.W. |
| (D) (bekannt) | k.W. |

Verbindungen gemäß Herstellungsbeispiel:

| | |
|---|---|
| I-2 | + + + + + |
| I-3 | + + + + + |
| I-4 | + + + + + |
| I-7 | + + + + + |
| I-9 | + + + + + |
| I-11 | + + + + + |
| I-13 | + + + + |
| I-14 | + + + + + |
| I-15 | + + + + + |
| I-16 | + + + + + |

Beispiel C

Antimikrobielle in-vivo-Wirksamkeit (lokal) am Modell der experimentellen Meerschweinchen-Trichophytie

Versuchsbeschreibung:

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert.

Die infizierten Tiere wurden beginnend mit dem 3. Tag p.i. 1x täglich mit einer 2,5 %igen Lösung der erfindungsgemäßen Präparate (in Dimethylsulfoxid : Glycerin = 1:4) lokal behandelt.

Ergebnis:

Bei unbehandelten Tieren entwickelte sich innerhalb von 12 Tagen p.i. das typische Bild einer Dermatophytose mit Rötung, Schuppung und Haarausfall bis zum totalen Integument-Defekt an der Infektionsstelle.

In diesem Test zeigen z. B. die erfindungsgemäßen Verbindungen (I-2) und (I-3) gute Wirkung.

Tabelle C

| Antimykotische in-vivo-Wirksamkeit (lokal) am Modell der experimentellen Meerschweinchen-Trichophytie | |
|---|---|
| Wirkstoff | Wirkung |
| Verbindung gemäß Herstellungsbeispiel: | |
| (I-2) | + + + + |
| (I-3) | + + + + |

Erläuterung:

| | | |
|---|---|---|
| + + + + + | = sehr gute Wirkung | = keine Infektionszeichen am 12. bis 15. Tag p.i. |
| + + + + | = gute Wirkung | = geringe Rötung, vereinzelt Schuppen |
| + + + | = Wirkung | = Rötung, Schuppen ohne Haarausfall |
| + + | = schwache Wirkung | = Rötung, Schuppen, Haarausfall |
| + | = Spur Wirkung | = flächiger Haarausfall, entzündliche Hautreaktion |

Beispiel D / Formulierungen

1.) Lösung:

| | |
|---|---|
| Wirkstoff gemäß Formel (I) | 10 g |
| Alkohol, rein (96 %ig) | 300 g |
| Isopropylmyristat | 526 g |
| | 836 g |

2.) Creme:

| | |
|---|---|
| Wirkstoff gemäß Formel (I) | 10 g |
| Aralcel 60 (Sorbitan-monostearat) | 20 g |
| Tween 60 (Polyoxyethylen (2) -sorbitan-monostearat) | 15 g |
| Walrat, künstlich (Mischung von Estern von gesättigten Fettsäuren $C_{14}$-$C_{18}$ und Fettalkoholen $C_{14}$-$C_{18}$) | 30 g |
| Lanette O | 100 g |
| Eutanol G (2-Octyl-dodecanol) | 135 g |
| Benzylalkohol | 10 g |
| Wasser, entmineralisiert | 680 g |
| | 1000 g |

**Patentansprüche**

**Patentansprüche für folgende Vertragsstaaten : AT, BE, CH, DE, FR, GB, GR, IT, LI, LU, NL, SE**

**1.** Azolylmethylcarbinole der allgemeinen Formel (I)

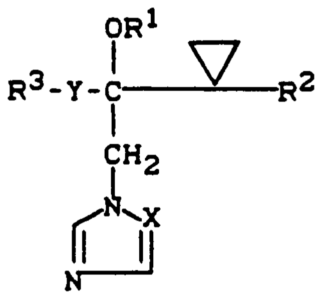

(I)

in welcher

$R^1$
- für Wasserstoff oder
- für Alkyl mit bis zu 10 Kohlenstoffatomen oder
- für Alkylcarbonyl mit bis zu 8 Kohlenstoffatomen steht,

$R^2$
- für Cyano oder
- für eine Gruppe der Formel

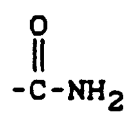

steht,

$R^3$
- für Phenyl steht, das gegebenenfalls bis zu 5-fach gleich oder verschieden substituiert ist durch Halogen, geradkettiges oder verzweigtes Alkyl mit bis zu 8 Kohlenstoffatomen, Alkoxy mit bis zu 6 Kohlenstoffatomen, Alkylthio mit bis zu 6 Kohlenstoffatomen,

Halogenalkyl, Halogenalkoxy oder Halogenalkylthio mit bis zu 6 Kohlenstoffatomen im Alkylteil und mit bis zu 5 Halogenatomen oder durch Phenyl oder Phenoxy, die ihrerseits durch Halogen oder Alkyl mit bis zu 6 Kohlenstoffatomen substituiert sein können,

X
- für ein Stickstoffatom oder für die CH-Gruppe steht,

Y
- für eine Bindung
- oder für eine Gruppe der Formel

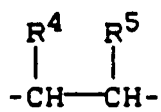,
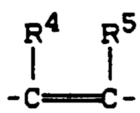

oder -C≡C- steht,

worin

$R^4$ und $R^5$ gleich oder verschieden sind und Wasserstoff oder Alkyl mit bis zu 8 Kohlenstoffatomen bedeuten,

sowie deren Säureadditions-Salze.

**2.** Azolylmethylcarbinole gemäß Anspruch 1, in welchen

$R^1$
- für Wasserstoff oder
- für Alkyl mit bis zu 8 Kohlenstoffatomen oder
- für Alkylcarbonyl mit bis zu 6 Kohlenstoffatomen steht,

$R^2$
- für Cyano oder für eine Gruppe der Formel

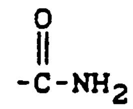

steht,

$R^3$
- für Phenyl steht, das gegebenenfalls bis zu 3-fach gleich oder verschieden substituiert ist durch Fluor, Chlor, Brom, geradkettiges oder verzweigtes Alkyl mit bis zu 6 Kohlenstoffatomen, Alkoxy mit bis zu 4 Kohlenstoffatomen, Alkylthio mit bis zu 4 Kohlenstoffatomen, Halogenalkyl, Halogenalkoxy oder Halogenalkylthio mit bis zu 4 Kohlenstoffatomen im Alkylteil und mit bis zu 4 Halogenatomen oder durch Phenyl oder Phenoxy, die ihrerseits durch Fluor, Chlor, Brom oder Alkyl mit bis zu 4 Kohlenstoffatomen substituiert sein können,

X
- für ein Stickstoffatom oder für die CH-Gruppe steht,

Y
- für eine Bindung
- oder für eine Gruppe der Formel

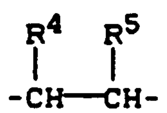,
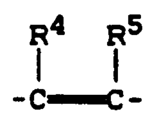

oder -C≡C- steht,

worin

$R^4$ und $R^5$ gleich oder verschieden sind und Wasserstoff oder Alkyl mit bis zu 6 Kohlenstoffatomen bedeuten,

sowie deren Säureadditions-Salze.

**3.** Azolylmethylcarbinole gemäß Anspruch 1, in welchen

$R^1$
- für Wasserstoff oder
- für Alkyl mit bis zu 6 Kohlenstoffatomen oder
- für Alkylcarbonyl mit bis zu 4 Kohlenstoffatomen steht,

$R^2$
- für Cyano oder für die Gruppe

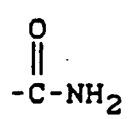

steht,

$R^3$
- für Phenyl steht, das gegebenenfalls bis zu 2-fach gleich oder verschieden substituiert ist durch Fluor, Chlor, Brom, Methyl, Ethyl, Isopropyl, tert.-Butyl, Methoxy, Methylthio, Trifluormethyl, Trifluormethoxy, Trifluormethylthio oder durch Phenyl oder Phenoxy, die ihrerseits durch Fluor, Chlor, Methyl oder Ethyl substituiert sein können,

X
- für ein Stickstoffatom oder für die CH-Gruppe steht,

Y
- für eine Bindung
- oder für eine Gruppe der Formel

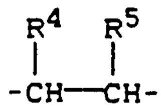 , 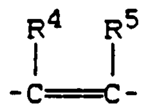

oder -C≡C- steht,
worin
$R^4$ und $R^5$ gleich oder verschieden sind und Wasserstoff, Methyl oder Ethyl bedeuten,
sowie deren Säureadditions-Salze.

**4.** Verfahren zur Herstellung von Azolylmethylcyclopropylcarbinolen gemäß Anspruch 1, indem man
[A] Verbindungen der allgemeinen Formel (II)

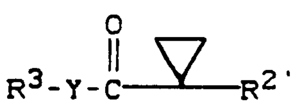 (II)

in welcher
$R^3$ und Y die in Anspruch 1 angegebene Bedeutung haben und
$R^{2'}$ für Cyano steht,
mit Sulfonium- oder Sulfoxoniumsalzen der Formel (III)

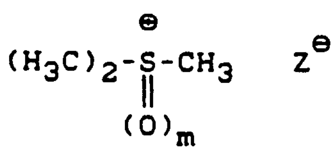 (III)

in welcher
Z - für Halogen steht und
m - eine Zahl 0 oder 1 bedeutet,
in einem inerten Lösungsmittel in Gegenwart einer Base bei Temperaturen von -20 °C bis 200 °C umsetzt, und die dabei entstehenden Oxirane der allgemeinen Formel (IV)

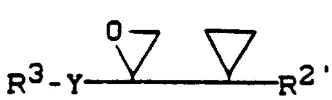 (IV)

in welcher
$R^3$ und Y die in Anspruch 1 angegebene Bedeutung haben und
$R^{2'}$ für Cyano steht mit Azolen der Formel (V)

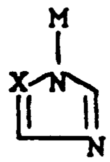

(V)

in welcher
X die in Anspruch 1 angegebene Bedeutung hat und
M für Wasserstoff oder für ein Alkali- oder Erdalkalimetallatom steht gegebenenfalls in einem inerten Lösungsmittel und in Anwesenheit einer Base zu Verbindungen der Formel (Ia)

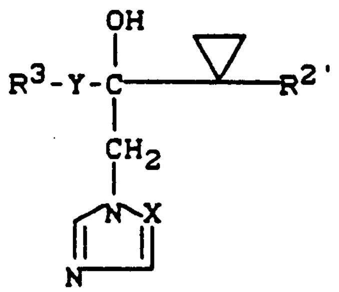

(Ia)

in welcher
$R^{2'}$, $R^3$, Y und X die oben angegebene Bedeutung haben, umsetzt,
oder indem man
[B] Verbindungen der allgemeinen Formel (Ia)

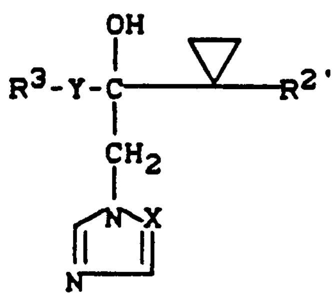

(Ia)

in welcher
$R^3$, X und Y die oben angegebene Bedeutung haben und
$R^{2'}$ - für Cyano steht
unter den Bedingungen einer Phasentransferreaktion in einem Zweiphasensystem, bestehend aus einer wäßrigen Lösung eines Alkalihydroxids und einem inerten organischen Lösungsmittel unter Zugabe von Wasserstoffperoxid und einem Katalysator bei Temperaturen von -20°C bis 60°C partiell zu Verbindungen der allgemeinen Formel (I), in welcher $R^{2'}$ für die Gruppe der Formel -CO-$NH_2$ steht, hydrolysiert,
oder indem man

[C] Azolylmethyl-cyclopropyl-carbinol-Derivate der Formel (Ib)

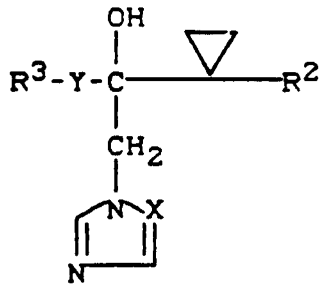

(Ib)

in welcher
$R^2$, $R^3$, X und Y die oben angegebene Bedeutung haben,
mit Basen in Gegenwart eines Verdünnungsmittels bei Temperaturen von 20 °C bis 100 °C umsetzt und die dabei entstehenden Alkoholate der allgemeinen Formel (Ic)

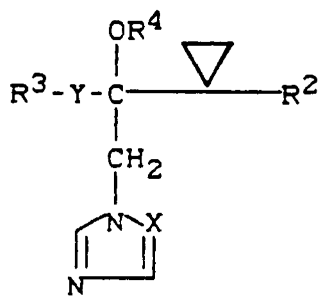

(Ic)

in welcher
$R^2$, $R^3$, X und Y die oben angegebene Bedeutung haben und
$R^4$ - für den kationischen Rest der Base steht
mit Verbindungen der Formel (VI)

$R^5$-W (VI)

in welcher
$R^5$ - für Alkyl mit bis zu 10 Kohlenstoffatomen, oder Acyl mit bis zu 8 Kohlenstoffatomen steht
W - für Halogen steht
in Gegenwart eines Verdünnungsmittels bei Temperaturen von 20 °C bis 100 °C umsetzt
und
gegebenenfalls anschließend an die so erhaltenen Verbindungen der allgemeinen Formel (I) in üblicher Weise eine Säure addiert und das Azolylmethylcarbinol der Formel (I) oder gegebenenfalls dessen Säureadditionssalz auf an sich bekannte Weise isoliert.

**5.** Verbindungen der allgemeinen Formel (II)

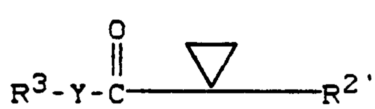

in der
$R^3$ und Y die in Anspruch 1 genannte Bedeutung haben und $R^{2'}$ für Cyano steht.

**6.** Verfahren zur Herstellung der Verbindungen gemäß Anspruch 5, in welchem $R^{2'}$, $R^3$ und Y die in Anspruch 5 angegebene Bedeutung haben, indem man N-Methyl-N-Methoxyamide der Formel (VII)

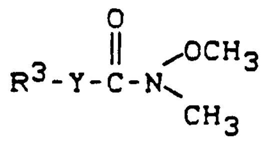

(VII)

in welcher
$R^3$ und Y die oben angegebene Bedeutung haben
mit dem Lithiumsalz von 1-Cyano-Cyclopropan der Formel (VIII)

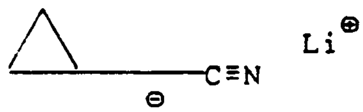

(VIII)

in einem inerten Lösungsmittel in einem Temperaturbereich von -100 °C bis +20 °C umsetzt.

**7.** Azolylmethylcarbinole gemäß Anspruch 1 zur Bekämpfung von Krankheiten.

**8.** Azolylmethylcarbinole gemäß Anspruch 1 zur Bekämpfung von Mykosen.

**9.** Arzneimittel enthaltend Azolylmethylcarbinole gemäß Anspruch 1.

**10.** Antimykotische Mittel enthaltend Azolylmethylcarbinole gemäß Anspruch 1.

**11.** Verwendung von Azolylmethylcarbinolen gemäß Anspruch 1 bei der Herstellung von Arzneimitteln.

**12.** Verwendung von Azolylmethylcarbinolen gemäß Anspruch 1 bei der Herstellung von Arzneimitteln zur Bekämpfung von Mykosen.

**Patentansprüche für folgenden Vertragsstaat : ES**

**1.** Verfahren zur Herstellung von Azolylmethylcyclopropylcarbinolen der Formel

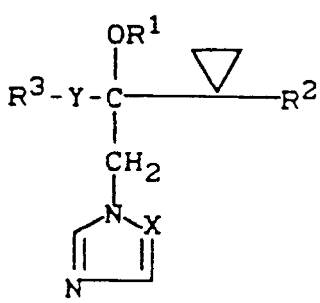

(I)

in welcher
$R^1$
- für Wasserstoff oder
- für Alkyl mit bis zu 10 Kohlenstoffatomen oder
- für Alkylcarbonyl mit bis zu 8 Kohlenstoffatomen steht,

$R^2$
- für Cyano oder

- für eine Gruppe der Formel

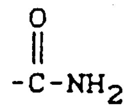

steht,

$R^3$

- für Phenyl steht, das gegebenenfalls bis zu 5-fach gleich oder verschieden substituiert ist durch Halogen, geradkettiges oder verzweigtes Alkyl mit bis zu 8 Kohlenstoffatomen, Alkoxy mit bis zu 6 Kohlenstoffatomen, Alkylthio mit bis zu 6 Kohlenstoffatomen, Halogenalkyl, Halogenalkoxy oder Halogenalkylthio mit bis zu 6 Kohlenstoffatomen im Alkylteil und mit bis zu 5 Halogenatomen oder durch Phenyl oder Phenoxy, die ihrerseits durch Halogen oder Alkyl mit bis zu 6 Kohlenstoffatomen substituiert sein können,

X

- für ein Stickstoffatom oder für die CH-Gruppe steht,

Y

- für eine Bindung
- oder für eine Gruppe der Formel

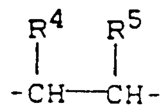 , 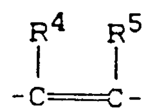

oder -C≡C- steht,

worin

$R^4$ und $R^5$ gleich oder verschieden sind und Wasserstoff oder Alkyl mit bis zu 8 Kohlenstoffatomen bedeuten,

sowie von deren Säureadditions-Salzen, dadurch gekennzeichnet, daß man

[A] Verbindungen der allgemeinen Formel (II)

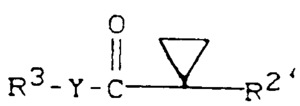 (II)

in welcher

$R^3$ und Y die oben angegebene Bedeutung haben und

$R^{2'}$ für Cyano steht,

mit Sulfonium- oder Sulfoxoniumsalzen der Formel (III)

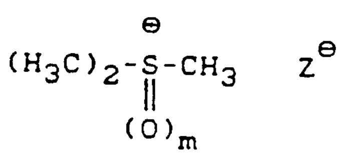 (III)

in welcher

Z - für Halogen steht und

m - eine Zahl 0 oder 1 bedeutet,

in einem inerten Lösungsmittel in Gegenwart einer Base bei Temperaturen von -20 °C bis 200 °C umsetzt, und die dabei entstehenden Oxirane der allgemeinen Formel (IV)

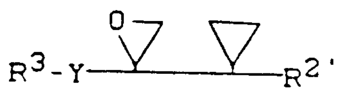

(IV)

in welcher

$R^3$ und Y die oben angegebene Bedeutung haben und

$R^{2'}$ für Cyano steht, mit Azolen der Formel (V)

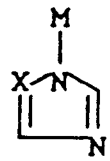

(V)

in welcher

X die oben angegebene Bedeutung hat und

M für Wasserstoff oder für ein Alkali- oder Erdalkalimetallatom steht, gegebenenfalls in einem inerten Lösungsmittel und in Anwesenheit einer Base zu Verbindungen der Formel (Ia)

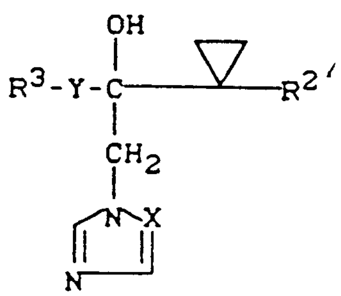

(Ia)

in welcher

$R^{2'}$, $R^3$, Y und X die oben angegebene Bedeutung haben, umsetzt,

oder daß man

[B] Verbindungen der allgemeinen Formel (Ia)

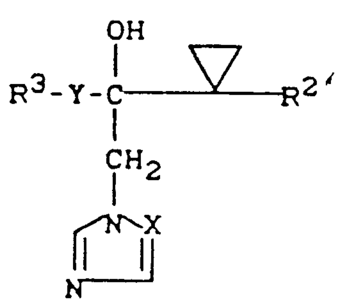

(Ia)

in welcher

$R^3$, X und Y die oben angegebene Bedeutung haben und

$R^{2'}$ - für Cyano steht

unter den Bedingungen einer Phasentransferreaktion in einem Zweiphasensystem, bestehend aus einer wäßrigen Lösung eines Alkalihydroxids und einem inerten organischen Lösungsmittel unter

Zugabe von Wasserstoffperoxid und einem Katalysator bei Temperaturen von -20 °C bis 60 °C partiell zu Verbindungen der allgemeinen Formel (I), in welcher $R^{2'}$ für die Gruppe der Formel $-CO-NH_2$ steht, hydrolysiert,
oder daß man
[C] Azolylmethyl-cyclopropyl-carbinol-Derivate der Formel (Ib)

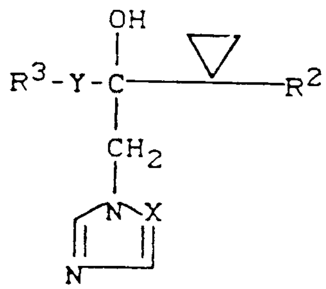
(Ib)

in welcher
$R^2$, $R^3$, X und Y die oben angegebene Bedeutung haben,
mit Basen in Gegenwart eines Verdünnungsmittels bei Temperaturen von 20 °C bis 100 °C umsetzt und die dabei entstehenden Alkoholate der allgemeinen Formel (Ic)

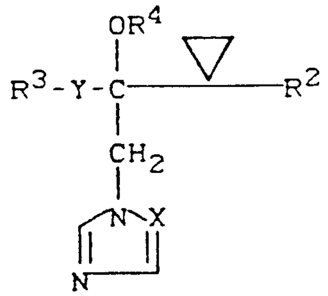
(Ic)

in welcher
$R^2$, $R^3$, X und Y die oben angegebene Bedeutung haben und
$R^4$ - für den kationischen Rest der Base steht,
mit Verbindungen der Formel (VI)

$R^5$-W (VI)

in welcher
$R^5$ - für Alkyl mit bis zu 10 Kohlenstoffatomen, oder Acyl mit bis zu 8 Kohlenstoffatomen steht
W - für Halogen steht
in Gegenwart eines Verdünnungsmittels bei Temperaturen von 20 °C bis 100 °C umsetzt
und
gegebenenfalls anschließend an die so erhaltenen Verbindungen der allgemeinen Formel (I) in üblicher Weise eine Säure addiert und das Azolylmethylcarbinol der Formel (I) oder gegebenenfalls dessen Säureadditionssalz auf an sich bekannte Weise isoliert.

**2.** Verfahren zur Herstellung von
Verbindungen der allgemeinen Formel (II)

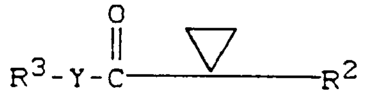

in der

$R^3$ und Y die in Anspruch 1 genannte Bedeutung haben und $R^{2'}$ für Cyano steht,

dadurch gekennzeichnet, daß man

N-Methyl-N-Methoxyamide der Formel (VII)

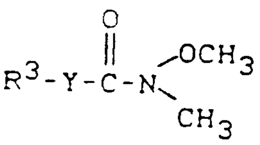

(VII)

in welcher

$R^3$ und Y die oben angegebene Bedeutung haben

mit dem Lithiumsalz von 1-Cyano-Cyclopropan der Formel (VIII)

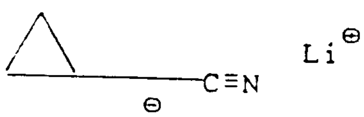

(VIII)

in einem inerten Lösungsmittel in einem Temperaturbereich von -100 °C bis +20 °C umsetzt.

**3.** Verwendung von Azolylmethylcarbinolen gemäß Anspruch 1 bei der Herstellung von Arzneimitteln.

**4.** Verwendung von Azolylmethylcarbinolen gemäß Anspruch 1 bei der Herstellung von Arzneimitteln zur Bekämpfung von Mykosen.

**Claims**

**Claims for the following Contracting States : AT, BE, CH, DE, FR, GB, GR, IT, LI, LU, NL, SE**

**1.** Azolylmethylcarbinols of the general formula (I)

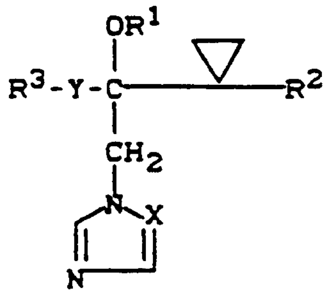

(I)

in which

$R^1$

- represents hydrogen or
- represents alkyl having up to 10 carbon atoms or
- represents alkylcarbonyl having up to 8 carbon atoms,

$R^2$

- represents cyano or

- represents a group of the formula

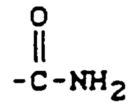

R³

- represents phenyl which is optionally monosubstituted to pentasubstituted by identical or different substituents from the series comprising halogen, straight-chain or branched alkyl having up to 8 carbon atoms, alkoxy having up to 6 carbon atoms, alkylthio having up to 6 carbon atoms, halogenoalkyl, halogenoalkoxy or halogenoalkylthio having up to 6 carbon atoms in the alkyl moiety and having up to 5 halogen atoms, or by phenyl or phenoxy which, in turn, may be substituted by halogen or alkyl having up to 6 carbon atoms,

X

- represents a nitrogen atom or the CH group,

Y

- represents a bond or
- represents a group of the formula

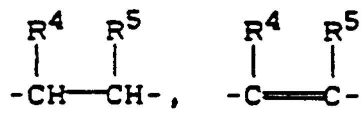

or -C≡C-

in which

$R^4$ and $R^5$ are identical or different and denote hydrogen or alkyl having up to 8 carbon atoms,

and their acid addition salts.

**2.** Azolylmethylcarbinols according to Claim 1, in which

$R^1$

- represents hydrogen or
- represents alkyl having up to 8 carbon atoms or
- represents alkylcarbonyl having up to 6 carbon atoms,

$R^2$

- represents cyano or a group of the formula

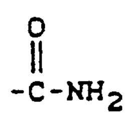

$R^3$

- represents phenyl which is optionally monosubstituted to trisubstituted by identical or different substituents from the series comprising fluorine, chlorine, bromine, straight-chain or branched alkyl having up to 6 carbon atoms,

alkoxy having up to 4 carbon atoms, alkylthio having up to 4 carbon atoms, halogenoalkyl, halogenoalkoxy or halogenoalkylthio having up to 4 carbon atoms in the alkyl moiety and having up to 4 halogen atoms, or by phenyl or phenoxy which, in turn, may be substituted by fluorine, chlorine, bromine or alkyl having up to 4 carbon atoms,

X

- represents a nitrogen atom or the CH group,

Y

- represents a bond or

- represents a group of the formula

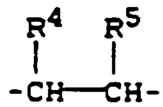, 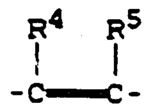

or -C≡C-

in which $R^4$ and $R^5$ are identical or different and denote hydrogen or alkyl having up to 6 carbon atoms,

and their acid addition salts.

**3.** Azolylmethylcarbinols according to Claim 1, in which

$R^1$
- represents hydrogen or
- represents alkyl having up to 6 carbon atoms or
- represents alkylcarbonyl having up to 4 carbon atoms,

$R^2$
- represents cyano or the group

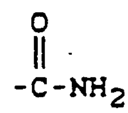

$R^3$
- represents phenyl which is optionally monosubstituted or disubstituted by identical or different substituents from the series comprising fluorine, chlorine, bromine, methyl, ethyl, isopropyl, tert.-butyl, methoxy, methylthio, trifluoromethyl, trifluoromethoxy, trifluoromethylthio or by phenyl or phenoxy which, in turn, may be substituted by fluorine, chlorine, methyl or ethyl,

X
- represents a nitrogen atom or the CH group,

Y
- represents a bond or
- represents a group of the formula

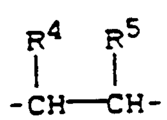, 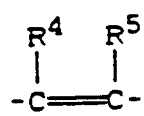

or -C≡C-

in which

$R^4$ and $R^5$ are identical or different and denote hydrogen, methyl or ethyl,

and their acid addition salts.

**4.** Process for the preparation of azolylmethylcyclopropylcarbinols according to Claim 1, by

[A] reacting compounds of the general formula (II) in which

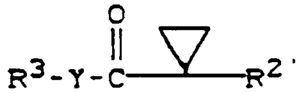 (II)

$R^3$ and Y have the meaning indicated in Claim 1 and
$R^{2'}$ represents cyano,

with sulphonium or sulphoxonium salts of the formula (III) in which

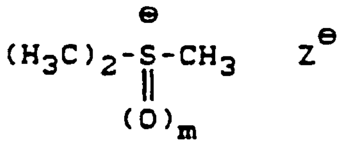

(III)

Z - represents halogen and
m - represents a number 0 or 1

in an inert solvent in the presence of a base at temperatures from -20 °C to 200 °C and reacting the oxiranes formed in this way of the general formula (IV)

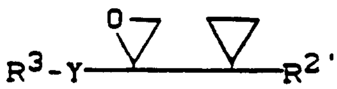

(IV)

in which
$R^3$ and Y have the meaning indicated in Claim 1 and
$R^{2'}$ represents cyano, with azoles of the formula (V)

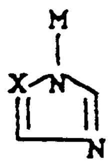

(V)

in which
X has the meaning indicated in Claim 1 and
M represents hydrogen or an alkali metal or alkaline earth metal atom, if appropriate in an inert solvent and in the presence of a base to give compounds of the formula (Ia)

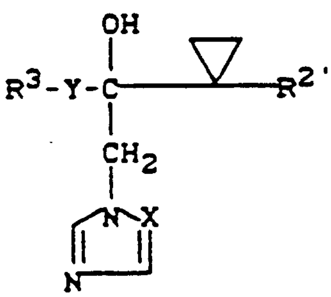

(Ia)

in which
$R^{2'}$, $R^3$, Y and X have the abovementioned meaning,
or by

[B] hydrolysing compounds of the general formula (Ia)

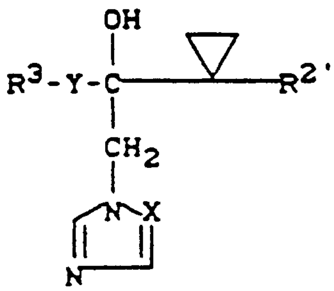

(Ia)

in which
$R^3$, X and Y have the abovementioned meaning and
$R^{2'}$ - represents cyano
under the conditions of a phase transfer reaction in a two-phase system consisting of an aqueous solution of an alkali metal hydroxide and an inert organic solvent with the addition of hydrogen peroxide and a catalyst, at temperatures from -20 °C to 60 °C partially to give compounds of the general formula (I) in which $R^{2'}$ represents the group of the formula $-CO-NH_2$,
or by
[C] reacting azolylmethylcyclopropylcarbinol derivatives of the formula (I b)

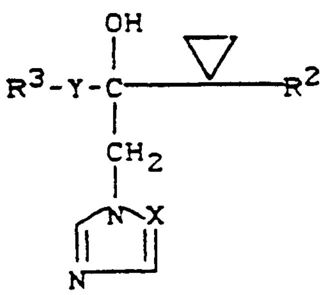

(Ib)

in which
$R^2$, $R^3$, X and Y have the abovementioned meaning, with bases in the presence of a diluent at temperatures from 20 °C to 100 °C and reacting the alkoxides formed in this way of the general formula (Ic)

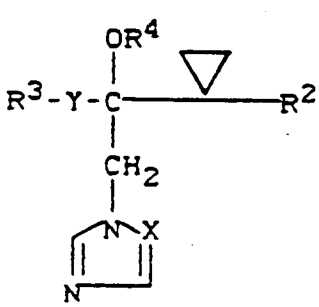

(Ic)

in which
$R^2$, $R^3$, X and Y have the abovementioned meaning and
$R^4$ - represents the cationic radical of a base
with compounds of the formula (VI)

$R^5$-W (VI)

in which
$R^5$
- represents alkyl having up to 10 carbon atoms, or acyl having up to 8 carbon atoms and

W
- represents halogen

in the presence of a diluent at temperatures from 20 °C to 100 °C
and
if appropriate subsequently adducting an acid in a customary manner to the compounds of the general formula (I) thus obtained and isolating the azolylmethylcarbinol of the formula (I) or, if desired, its acid addition salt in a manner known per se.

**5.** Compounds of the general formula (II)

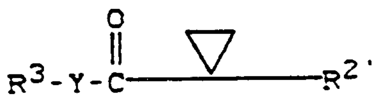

in which
$R^3$ and Y   have the meaning mentioned in Claim 1 and $R^{2'}$ represents cyano.

**6.** Process for the preparation of the compounds according to Claim 5, in which $R^{2'}$, $R^3$ and Y have the meaning indicated in Claim 5 by reacting N-methyl-N-methoxyamides of the formula (VII)

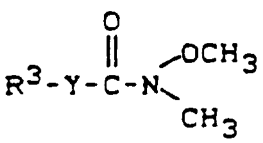

(VII)

in which
$R^3$ and Y   have the abovementioned meaning,
with the lithium salt of 1-cyanocyclopropane of the formula (VIII)

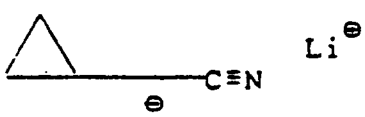

(VIII)

in an inert solvent in a temperature range from -100 °C to +20 °C.

**7.** Azolylmethylcarbinols according to Claim 1 for combating diseases.

**8.** Azolylmethylcarbinols according to Claim 1 for combating mycoses.

**9.** Medicaments containing azolylmethylcarbinols according to Claim 1.

**10.** Antimycotic agents containing azolylmethylcarbinols according to Claim 1.

**11.** Use of azolymethylcarbinols according to Claim 1 in the production of medicaments.

**12.** Use of azolymethylcarbinols according to Claim 1 in the production of medicaments for combating mycoses.

**Claims for the following Contracting State : ES**

**1.** Process for the preparation of azolylmethylcyclopropylcarbinols of the formula

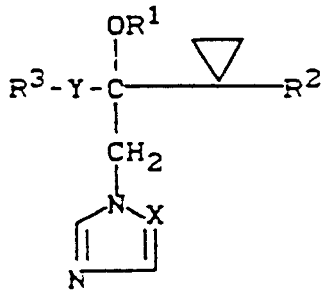

(I)

in which

$R^1$

- represents hydrogen or
- represents alkyl having up to 10 carbon atoms or
- represents alkylcarbonyl having up to 8 carbon atoms,

$R^2$

- represents cyano or
- represents a group of the formula

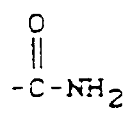

$R^3$

- represents phenyl which is optionally monosubstituted to pentasubstituted by identical or different substituents from the series comprising halogen, straight-chain or branched alkyl having up to 8 carbon atoms, alkoxy having up to 6 carbon atoms, alkylthio having up to 6 carbon atoms, halogenoalkyl, halogenoalkoxy or halogenoalkylthio having up to 6 carbon atoms in the alkyl moiety and having up to 5 halogen atoms, or by phenyl or phenoxy which, in turn, may be substituted by halogen or alkyl having up to 6 carbon atoms,

X

- represents a nitrogen atom or the CH group,

Y

- represents a bond or
- represents a group of the formula

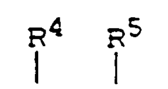 , 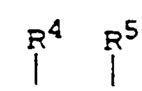

or -C≡C-

in which

$R^4$ and $R^5$ are identical or different and denote hydrogen or alkyl having up to 8 carbon atoms,

and of their acid addition salts,

characterized in that

[A] compounds of the general formula (II)

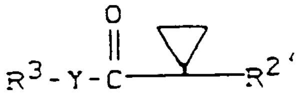

(II)

in which

$R^3$ and Y have the meaning indicated above and

$R^{2'}$ represents cyano,

are reacted with sulphonium or sulphoxonium salts of the formula (III)

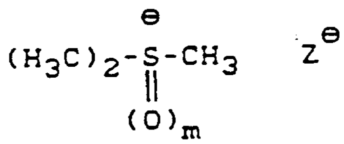

(III)

in which

Z - represents halogen and

m - represents a number 0 or 1

in an inert solvent in the presence of a base at temperatures from -20 °C to 200 °C and the oxiranes formed in this way of the general formula (IV)

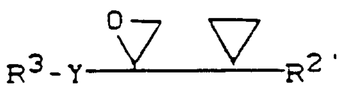

(IV)

in which

$R^3$ and Y have the abovementioned meaning indicated and

$R^{2'}$ represents cyano, are reacted with azoles of the formula (V)

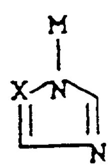

(V)

in which

X has the abovementioned meaning indicated and

M represents hydrogen or an alkali metal or alkaline earth metal atom, if appropriate in an inert solvent and in the presence of a base to give compounds of the formula (Ia)

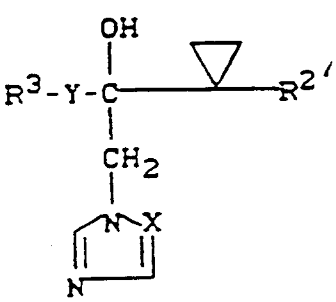

(Ia)

in which
$R^{2'}$, $R^3$, Y and X have the abovementioned meaning,
or in that
[B] compounds of the general formula (Ia)

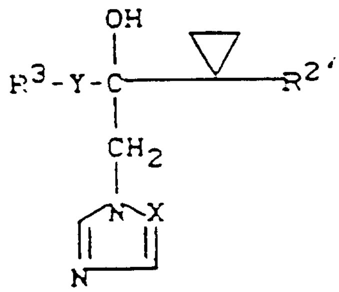

(Ia)

in which
$R^3$, X and Y have the abovementioned meaning and
$R^{2'}$ - represents cyano
are hydrolysed under the conditions of a phase transfer reaction in a two-phase system consisting of an aqueous solution of an alkali metal hydroxide and an inert organic solvent with the addition of hydrogen peroxide and a catalyst, at temperatures from -20 °C to 60 °C partially to give compounds of the general formula (I) in which $R^{2'}$ represents the group of the formula $-CO-NH_2$,
or in that
[C] azolylmethylcyclopropylcarbinol derivatives of the formula (I b)

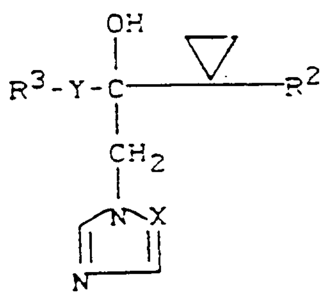

(Ib)

in which
$R^2$, $R^3$, X and Y have the abovementioned meaning,
are reacted with bases in the presence of a diluent at temperatures from 20 °C to 100 °C and the alkoxides formed in this way of the general formula (Ic)

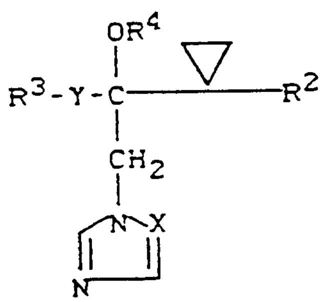

(Ic)

in which
$R^2$, $R^3$, X and Y have the abovementioned meaning and
$R^4$ - represents the cationic radical of a base,
are reacted with compounds of the formula (VI)

$R^5$-W (VI)

in which

$R^5$
- represents alkyl having up to 10 carbon atoms, or acyl having up to 8 carbon atoms and

W
- represents halogen

in the presence of a diluent at temperatures from 20 °C to 100 °C
and
if appropriate an acid is subsequently adducted in a customary manner to the compounds of the general formula (I) thus obtained and the azolylmethylcarbinol of the formula (I) or, if desired, its acid addition salt is isolated in a manner known per se.

**2.** Process for the preparation of compounds of the general formula (II)

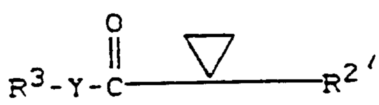

in which

$R^3$ and Y have the meaning mentioned in Claim 1 and $R^{2'}$ represents cyano,

characterized in that

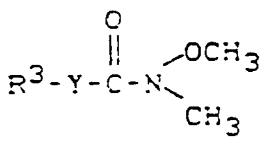

(VII)

in which

$R^3$ and Y have the abovementioned meaning,

are reacted with the lithium salt of 1-cyanocyclopropane of the formula (VIII)

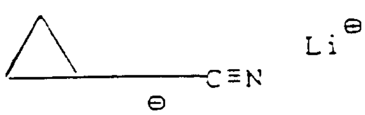

(VIII)

in an inert solvent in a temperature range from -100 °C to +20 °C.

**3.** Use of azolymethylcarbinols according to Claim 1 in the production of medicaments.

**4.** Use of azolymethylcarbinols according to Claim 1 in the production of medicaments for combating mycoses.

**Revendications**

**Revendications pour les Etats contractants suivants : AT, BE, CH, DE, FR, GB, GR, IT, LI, LU, NL, SE**

**1.** Azolylméthylcarbinols de formule générale (I)

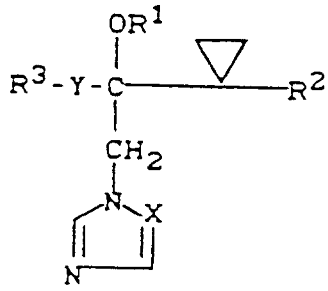

(I)

dans laquelle

$R^1$
- représente l'hydrogène ou
- un groupe alkyle ayant jusqu'à 10 atomes de carbone ou
- un groupe alkylcarbonyle ayant jusqu'à 8 atomes de carbone,

$R^2$
- est un groupe cyano ou
- un groupe de formule

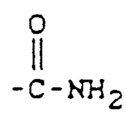

$R^3$
- est un groupe phényle qui est substitué jusqu'à 5 fois identiques ou différentes par un halogène, un groupe alkyle à chaîne droite ou ramifiée ayant jusqu'à 8 atomes de carbone, un groupe alkoxy ayant jusqu'à 6 atomes de carbone, un groupe alkylthio ayant jusqu'à 6 atomes de carbone, un groupe halogénalkyle, halogénalkoxy ou halogénalkylthio ayant jusqu'à 6 atomes de carbone dans la partie alkyle et jusqu'à 5 atomes d'halogènes, ou par un groupe phényle ou un groupe phénoxy qui peuvent être substitués quant à eux par un halogène ou un radical alkyle ayant jusqu'à 6 atomes de carbone,

X
- représente un atome d'azote ou le groupe CH-,

Y
- est une liaison
- ou bien un groupe de foule

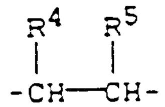 , 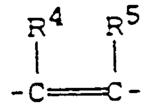

ou -C≡C-

dans laquelle

$R^4$ et $R^5$ sont identiques ou différents et représentent de l'hydrogène ou des groupes alkyle ayant jusqu'à 8 atomes de carbone,

ainsi que leurs sels d'addition d'acides.

2. Azolylméthylcarbinols suivant la revendication 1, dans lesquels

$R^1$
- représente l'hydrogène ou
- un groupe alkyle ayant jusqu'à 8 atomes de carbone ou
- un groupe alkylcarbonyle ayant jusqu'à 6 atomes de carbone,

$R^2$
- est un groupe cyano ou un groupe de formule

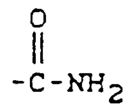

$R^3$
- est un groupe phényle qui est substitué le cas échéant jusqu'à 3 fois identiques ou différentes par du fluor, du chlore, du brome, un radical alkyle à chaîne droite ou ramifiée ayant jusqu'à 6 atomes de carbone, alkoxy ayant jusqu'à 4 atomes de carbone, alkylthio ayant jusqu'à 4 atomes de carbone, halogénalkyle, halogénalkoxy ou halogénalkylthio ayant jusqu'à 4 atomes de carbone dans la partie alkyle et jusqu'à 4 atomes d'halogènes ou par un groupe phényle ou un groupe phénoxy qui peuvent être substitués quant à eux par du fluor, du chlore, du brome ou un radical alkyle ayant jusqu'à 4 atomes de carbone,

X
- représente un atome d'azote ou le groupe CH-,

Y
- est une liaison
- ou bien un groupe de formule

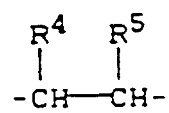,
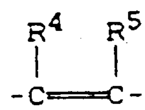

ou -C≡C-

dans laquelle

$R^4$ et $R^5$ sont identiques ou différents et représentent de l'hydrogène ou un groupe alkyle ayant jusqu'à 6 atomes de carbone,

ainsi que leurs sels d'addition d'acides.

3. Azolylméthylcarbinols suivant la revendication 1, dans lesquels

$R^1$
- représente l'hydrogène ou
- un groupe alkyle ayant jusqu'à 6 atomes de carbone ou
- un groupe alkylcarbonyle ayant jusqu'à 4 atomes de carbone,

$R^2$
- est un groupe cyano ou le groupe

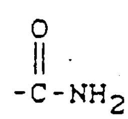

$R^3$
- est un groupe phényle qui est substitué le cas échéant jusqu'à 2 fois identiques ou différentes par du fluor, du chlore, du brome, un radical méthyle, éthyle,

isopropyle, tertio-butyle, méthoxy, méthylthio, trifluorométhyle, trifluorométhoxy, trifluorométhylthio ou par un groupe phényle ou un groupe phénoxy qui peuvent être substitués quant à eux par du fluor, du chlore, un radical méthyle ou éthyle,

X
- représente un atome d'azote ou le groupe CH-,

Y
- représente une liaison
- ou un groupe de formule

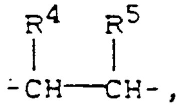 , 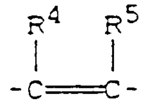

ou -C≡C-

dans laquelle

$R^4$ et $R^5$ sont identiques ou différents et représentent l'hydrogène, un radical méthyle ou éthyle,

ainsi que leurs sels d'addition d'acides.

**4.** Procédé de production d'azolylméthylcyclopropylcarbinols suivant la revendication 1, dans lequel

[A] On fait réagir des composés de formule générale (II)

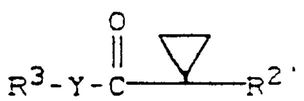 (II)

dans laquelle

$R^3$ et Y ont la définition indiquée dans la re-revendication 1 et

$R^{2'}$ est un groupe cyano,

avec des sels de sulfonium ou de sulfoxonium de formule (III)

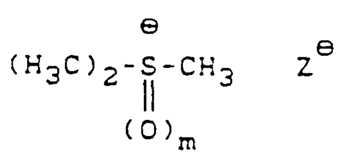 (III)

dans laquelle

Z - représente un halogène et

m - est le nombre 0 ou 1,

dans un solvant inerte en présence d'une base à des températures allant de -20 °C à 200 °C et on fait réagir les oxirannes ainsi produits, de formule générale (IV)

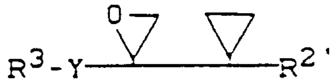 (IV)

dans laquelle

$R^3$ et Y ont la définition indiquée dans la revendication 1 et

$R^{2'}$ est un groupe cyano, avec des azoles de formule (V)

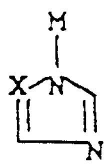

(V)

dans laquelle

X a la définition indiquée dans la revendication 1 et

M représente l'hydrogène ou un atome de métal alcalin ou de métal alcalino-terreux, le cas échéant dans un solvant inerte et en présence d'une base pour former des composés de formule (Ia)

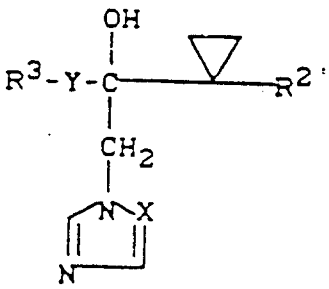

(Ia)

dans laquelle

$R^{2'}$, $R^3$, Y et X ont la définition indiquée ci-dessus,

ou bien

[B] On hydrolyse partiellement des composés de formule générale (Ia)

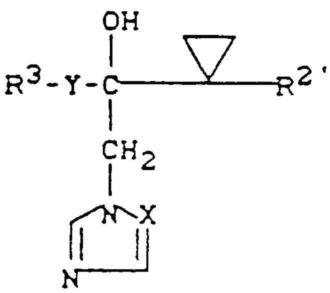

(Ia)

dans laquelle

$R^3$, X et Y ont la définition indiquée ci-dessus et

$R^{2'}$ - est un groupe cyano,

dans les conditions d'une réaction de transfert de phase dans un système de deux phases constitué d'une solution aqueuse d'hydroxyde de métal alcalin et d'un solvant organique inerte, avec addition de peroxyde d'hydrogène et d'un catalyseur à des températures allant de -20°C à 60°C en composés de formule générale (I) dans laquelle $R^{2'}$ représente le groupe de formule $-CO-NH_2$,

ou bien

[c] On fait réagir des dérivés d'azolylméthylcyclopropylcarbinols de formule (Ib)

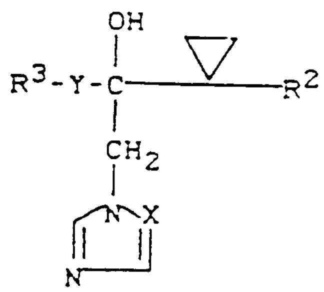

(Ib)

dans laquelle
$R^2$, $R^3$, X et Y ont la définition indiquée ci-dessus
avec des bases en présence d'un diluant à des températures allant de 20°C à 100°C et on fait réagir les alcoolates ainsi produits de formule générale (Ic)

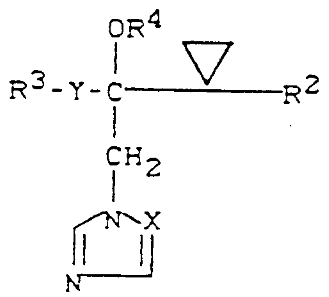

(Ic)

dans laquelle
$R^2$, $R^3$, X et Y ont la définition indiquée ci-dessus et
$R^4$ - est le reste cationique de la base,
avec des composés de formule (VI)

$R^5$-W (VI)

dans laquelle
$R^5$
- est un groupe alkyle ayant jusqu'à 10 atomes de carbone ou un groupe acyle ayant jusqu'à 8 atomes de carbone
W
- est un halogène,
en présence d'un diluant à des températures de 20°C à 100°C
et
le cas échéant, on additionne ensuite un acide de manière classique sur les composés de formule générale (I) ainsi obtenus et on isole de manière connue l'azolylméthylcarbinol de formule (I) ou le cas échéant son sel d'addition d'acide.

**5.** Composés de formule générale (II)

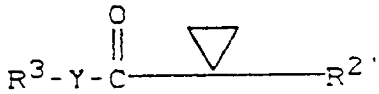

dans laquelle

$R^3$ et Y ont la définition indiquée dans la revendication 1 et $R^{2'}$ est un groupe cyano.

6. Procédé de production des composés suivant la revendication 4, dans lequel $R^{2'}$, $R^3$ et Y ont la définition indiquée dans la revendication 4, dans lequel on fait réagir des N-méthyl-N-méthoxyamides de formule (VII)

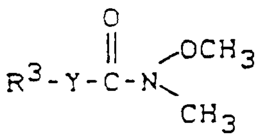

(VII)

dans laquelle
$R^3$ et Y ont la définition indiquée ci-dessus
avec le sel de lithium du 1-cyano-cyclopropane de formule (VIII)

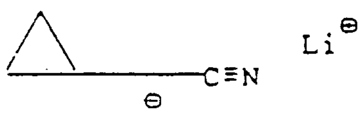

(VIII)

dans un solvant inerte, dans une plage de températures allant de -100 °C à +20 °C.

7. Azolylméthylcarbinols suivant la revendication 1, destinés à combattre des maladies.

8. Azolylméthylcarbinols suivant la revendication 1, destinés à combattre des mycoses.

9. Médicaments contenant des azolylméthylcarbinols suivant la revendication 1.

10. Compositions anti-mycosiques contenant des azolylméthylcarbinols suivant la revendication 1.

11. Utilisation d'azolylméthylcarbinols suivant la revendication 1 pour la préparation de médicaments.

12. Utilisation d'azolylméthylcarbinols suivant la revendication 1 pour la préparation de médicaments destinés à combattre des mycoses.

**Revendications pour l'Etat contractant suivant : ES**

1. Procédé de production d'azolylméthylcyclopropylcarbinols de formule

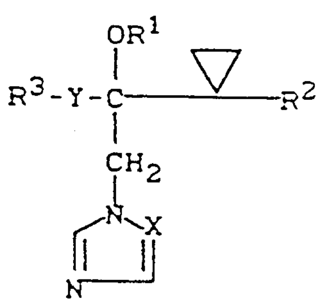

(I)

dans laquelle
$R^1$
- représente l'hydrogène ou

- un groupe alkyle ayant jusqu'à 10 atomes de carbone ou
- un groupe alkylcarbonyle ayant jusqu'à 8 atomes de carbone,

$R^2$

- est un groupe cyano ou
- un groupe de formule

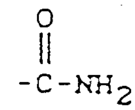

$R^3$

- est un groupe phényle qui est substitué jusqu'à 5 fois identiques ou différentes par un halogène, un groupe alkyle à chaîne droite ou ramifiée ayant jusqu'à 8 atomes de carbone, un groupe alkoxy ayant jusqu'à 6 atomes de carbone, un groupe alkylthio ayant jusqu'à 6 atomes de carbone, un groupe halogénalkyle, halogénalkoxy ou halogénalkylthio ayant jusqu'à 6 atomes de carbone dans la partie alkyle et jusqu'à 5 atomes d'halogènes, ou par un groupe phényle ou un groupe phénoxy qui peuvent être substitués quant à eux par un halogène ou un radical alkyle ayant jusqu'à 6 atomes de carbone,

X

- représente un atome d'azote ou le groupe CH-,

Y

- est une liaison
- ou bien un groupe de formule

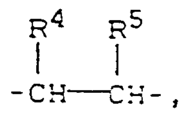 , 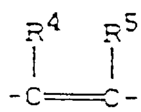

ou $-C \equiv C-$

dans laquelle

$R^4$ et $R^5$ sont identiques ou différents et représentent de l'hydrogène ou des groupes alkyle ayant jusqu'à 8 atomes de carbone,

ainsi que de leurs sels d'addition d'acides,
caractérisé en ce que

[A] On fait réagir des composés de formule générale (II)

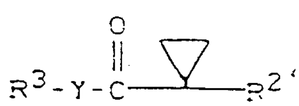 (II)

dans laquelle

$R^3$ et Y ont la définition indiquée ci-dessus et
$R^{2'}$ est un groupe cyano,
avec des sels de sulfonium ou de sulfoxonium de formule (III)

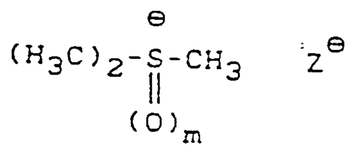

(III)

dans laquelle

Z - est un halogène et

m - a la valeur 0 ou 1,

dans un solvant inerte en présence d'une base à des températures allant de -20 °C à 200 °C et on fait réagir les oxirannes ainsi produits de formule générale (IV)

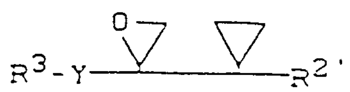

(IV)

dans laquelle

$R^3$ et Y ont la définition indiquée ci-dessus et

$R^{2'}$ est un groupe cyano, avec des azoles de formule (V)

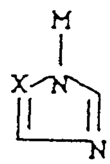

(V)

dans laquelle

X a la définition indiquée ci-dessus et

M représente l'hydrogène ou un atome de métal alcalin ou de métal alcalino-terreux, le cas échéant dans un solvant inerte et en présence d'une base pour former des composés de formule (Ia)

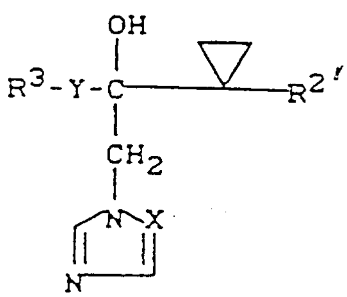

(Ia)

dans laquelle

$R^{2'}$, $R^3$, Y et X ont la définition indiquée ci-dessus,

ou bien

[B] On hydrolyse partiellement des composés de formule générale (Ia)

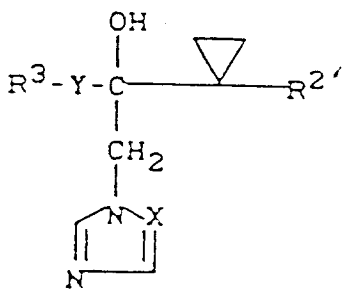

(Ia)

dans laquelle
$R^3$, X et Y ont la définition indiquée ci-dessus et
$R^{2'}$ - est un groupe cyano,
dans les conditions d'une réaction de transfert de phase dans un système de deux phases constitué d'une solution aqueuse d'un hydroxyde alcalin et d'un solvant organique inerte, avec addition de peroxyde d'hydrogène et d'un catalyseur, à des températures allant de -20 °C à 60 °C pour former des composés de formule générale (I) dans laquelle $R^{2'}$ représente le groupe de formule $-CO-NH_2$, ou bien
[C] On fait réagir des dérivés d'azolylméthylcyclopropylcarbinols de formule (Ib)

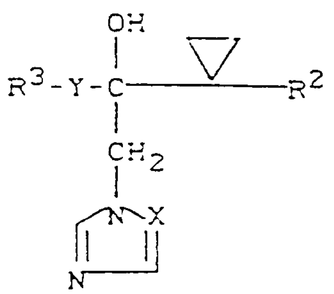

(Ib)

dans laquelle
$R^2$, $R^3$, X et Y ont la définition indiquée ci-dessus
avec des bases en présence d'un diluant à des températures allant de 20 °C à 100 °C et on fait réagir les alcoolates ainsi produits de formule générale (Ic)

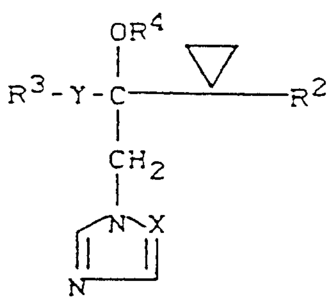

(Ic)

dans laquelle
$R^2$, $R^3$, X et Y ont la définition indiquée ci-dessus et
$R^4$ - est le reste cationique de la base,
avec des composés de formule (VI)

$R^5$-W (VI)

dans laquelle

$R^5$
- est un groupe alkyle ayant jusqu'à 10 atomes de carbone ou un groupe acyle ayant jusqu'à 8 atomes de carbone

W
- représente un halogène,

en présence d'un diluant à des températures de 20 °C à 100 °C
et
le cas échéant, on additionne ensuite un acide de manière classique sur les composés de formule générale (I) ainsi obtenus et on isole l'azolylméthylcarbinol de formule (I) ou le cas échéant son sel d'addition d'acide, d'une manière connue.

**2.** Procédé de production de composés de formule générale (II)

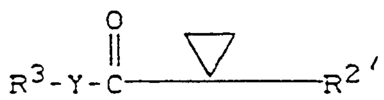

dans laquelle
$R^3$ et Y ont la définition indiquée dans la revendication 1 et $R^{2'}$ est un groupe cyano,
caractérisé en ce qu'on fait réagir des N-méthyl-N-méthoxyamides de formule (VII)

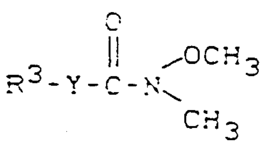

(VII)

dans laquelle
$R^3$ et Y ont la définition indiquée ci-dessus
avec le sel de lithium du 1-cyano-cyclopropane de formule (VIII)

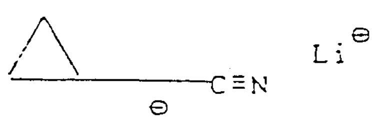

(VIII)

dans un solvant inerte, dans une plage de températures de -100 °C à +20 °C.

**3.** Utilisation d'azolylméthylcarbinols suivant la revendication 1 dans la préparation de médicaments.

**4.** Utilisation d'azolylméthylcarbinols suivant la revendication 1 dans la préparation de médicaments destinés à combattre des mycoses.